# EUROPEAN PATENT SPECIFICATION

(11) **EP 0 519 596 B1**
(45) Date of publication and mention of the grant of the patent: **23.02.2005**
(21) Application number: 92304225.3
(22) Date of filing: 12.05.1992
(51) Int. Cl.: C12N 15/13, C12P 21/08

(54) **A method for reducing the immunogenicity of antibody variable domains**
Verfahren zur Verminderung der Immunogenität der variablen Antikörperdomänen
Procédé pour réduire l'immunogénicité des domaines variables d'anticorps

(30) Priority: 17.05.1991 US 702217
(43) Date of publication of application: 23.12.1992
(73) Proprietor: Merck & Co., Inc., Rahway New Jersey 07065-0900 (US); NATIONAL INSTITUTES OF HEALTH, Bethesda, Maryland 20892 (US)
(72) Inventor: Padlan, Eduardo A., Kensington, Maryland 20895 (US); Mark, George E., III, Princeton Junction, NJ 085550 (US); Daugherty, Bruce L., S. Orange, NJ 07079 (US)
(74) Representative: Horgan, James Michael Frederic

(56) References cited:
- EP-A- 0 438 312
- WO-A-90/07861
- PROCEEDINGS OF THE NATIONAL ACADEMY OF SCIENCES OF USA. vol. 88, no. 10, 15 May 1991, WASHINGTON DC, US pages 4181 - 4185 S. GORMAN ET AL. 'Reshaping a therapeutic CD4 antibody.'
- NUCLEIC ACIDS RESEARCH vol. 19, no. 9, 11 May 1991, LONDON, GB pages 2471 - 2476 B. DAUGHERTY ET AL. 'Polymerase chain reaction facilitates the cloning, CDR-grafting, and rapid expression of a murine monoclonal antibody directed against the CD18 component of leukocyte integrins.'
- NATURE. vol. 332, 24 March 1988, LONDON, GB pages 323 - 327 L. RIECHMANN ET AL. 'Reshaping human antibodies for therapy.'
- Padlan E.A., Proteins: Structure, Function and Genetics, 7: 112-124 (1990)
- Tempest P.R. et al.: Bio/Technology, 9: 266-271 (March 1991)

## Description

### BRIEF DESCRIPTION OF THE DRAWINGS

Figure 1. Solvent exposure of sidechains of framework residues in KOL and J539 Fvs and the residues which occur most frequently at these positions in the various human VH subgroups.
Figure 2. Solvent exposure of sidechains of framework residues in KOL VL and the residues which occur most frequently at these positions in the various human V-lambda subgroups.
Figure 3. Solvent exposure of sidechains of framework residues in J539 VL and the residues which occur most frequently at these positions in the various human V-kappa subgroups.

Figure 4. Primers used to isolate DNA encoding murine kappa light chain variable region and murine IgG2a heavy chain variable region using PCR. Oligodeoxynucleotides used as PCR primers to generate a shortened IgG4 heavy chain. Oligodeoxynucleotides used in PCR to re-engineer the thymidine kinase (TK) promoter to facilitate the expression of the neomycin resistance gene. Oligodeoxynucleotide primers used in PCR to clone the IgH enhancer sequence. Oligodeoxynucleotides used as PCR primers to generate a human kappa light chain constant region.

Figure 5. Oligodeoxynucleotides used in the construction of the "veneered" 1B4 heavy and light chain variable regions plus those necessary to fuse the human signal and intronic sequenceds onto these variable regions.

Figure 6. PCR-recombination strategy used in the veneering of the 1B4 kappa light chain variable region.

Figure 7. Outline of the insertion of the "veneered" kappa light chain variable region and kappa constant region into the light chain expression vector.

Figure 8. PCR-recombination strategy used in the veneering of the 1B4 heavy chain variable region.

Figure 9. Outline of the insertion of the "veneered" heavy chain variable region into the heavy chain expression vector.

Figure 10. Outline of the construction of neomycin selectable expression vector.

Figure 11. Outline of the construction of the hygromycin selectable expression vector.

Figure 12. Amino acid sequence completion of the "veneered"-1B4, murine 1B4 and human Gal heavy chain variable regions and the "veneered" 1B4, murine 1B4 and human Len kappa light chain variable regions. Check marks indicate the individual amino acid residues converted.

Figure 13. Competitive binding assay of native murine 1B4 (open diamonds) and recombinant "veneered" 1B4 (closed diamonds).

### BACKGROUND OF THE INVENTION

The identification and production of murine monoclonal antibodies has lead to numerous therapeutic applications of these exquisitely specific molecules in human disease. The technologies of molecular biology have further expanded the utility of many antibodies by allowing for the creation of class switched molecules whose functionality has been improved by the acquisition or loss of complement fixation. The size of the bioactive molecule may also be reduced so as to increase the tissue target availability of the antibody by either changing the class from an IgM to an IgG, removing most of the heavy chain constant region in the creation of a F(ab)₂ or both heavy and light chain constant regions may be dispensed with in the formation of a Fv antibody. Common to all of these potentially therapeutic forms of antibody are the requisite CDRs (complementary determining regions) which guide the molecule to its ligand and the framework residues (FRs) which support these latter structures and dictate the disposition of the CDRs relative to one another, Winter European Patent Application, Publication No. 239,400; Riechmann et al., Nature 332: 323-327 (1988). Crystallographic analyses of numerous antibody structures reveal that the combining site is composed almost entirely of the CDR residues arranged in a limited number of loop motifs, Padlan and Sheriff, 1990. The necessity of the CDRs to form these structures combined with the appreciated hypervariablity of their primary sequence leads to a great diversity in the antigen combining site, but one which has a finite number of possibilities. Thus, hypermutability and a limited primary sequence repetoire for each CDR would suggest that the CDRs derived for a given antigen from one species of animal would be the same derived from another species. Hence, they should be poorly immunogenic, if at all, when presented to a recipient organism in a non-foreign context.

Monoclonal antibody producing hybridomas have been most readily obtained from immunized rodents. Development of similar reagents from human sources has been frustrated by the current inability to maintain long term cultures of cells which produce sufficient quantities of antibody. Additional problems arise from the regulatory standpoint when cells of human origin are employed for the production of agents to be used in man. These considerations have lead to the widespread use of rodent monoclonal antibodies for the imaging and treatment of malignancy, prophylactic administration to guard against toxic shock, modification of graft rejection episodes, and to temper acute inflammatory reactions. In all scenarios where completely rodent or partially rodent (ie, rodent - human chimeras) antibodies have been used for therapy the recipients have often elicited an immune response directed toward the antibody. These reactions have limited the duration and effectiveness of the therapy.

Various attempts have been made to minimize or eliminate the immunogenicity of non-human antibodies while perserving their antigen- binding properties. Initially, chimeric antibodies were constructed containing the rodent varible regions and their associated CDRs fused to human constant domains. The following references generally describe chimaeric antibody technology: Lobuglio et al., Proc. Natl. Acad. Sci. USA 86: 4220-4224 (1989); United States Patent 4,816,567; PCT International Publication No. WO 87/02671, published May 7,1987; European Patent Publication No. 255,694, published February 10 1988; European Patent Publication No. 274,394, published July 13, 1988; European Patent Publication No. 323,806, published July 12, 1989; PCT International Publication No. WO/89/00999, published February 9, 1989; European Patent Publication No. 327,000, published August 9, 1989; European Patent Publication No. 328,404, published August 16,1989; and European Patent Publication No. 332,424, published September 13, 1989. These proved to be less immunogenic but still approximately half of the recipients mounted an immune response to the rodent variable region framework residues. Further reduction of the "foreign" nature of the chimeric antibodies has been achieved by grafting only the CDRs from the rodent monoclonal into a human supporting framework prior to its subsequent fusion with an appropriate constant domain, Winter European Patent Application, Publication No. 239,400; Riechmann et al., Nature 332: 323-327 (1988). The procedures employed to accomplish CDR-grafting often result in imperfectly "humanized" antibodies. That is to say, the resultant antibody has either lost avidity (usually 2-3 fold, at best) or in an attempt to retain its original avidity a significant number of the murine framework residues have replaced the corresponding ones of the chosen human framework. In this later case, the immunogenicity of the modified "humanized" antibody is difficult to anticipate a priori.

The ligand binding characteristics of an antibody combining site are determined primarily by the structure and relative disposition of the CDRs, although some neighboring residues also have been found to be involved in antigen binding (Davies et al., Ann. Rev. Biochem. 59: 439-473 [1990]). Fine specificity can be perserved in a "humanized" antibody only if the CDR structures, their interaction with each other, and their interaction with the rest of the variable domains are strictly maintained. One may anticipate that the key residues represent "interior" and interdomain contact residues, hence those surface exposed residues which are immediately available for immune surveillance should be non- inclusive of the structural residues.

EP-A-438312 discloses the incorporation of a CDR from a first species into a second species which has similar variable heavy and light chain frameworks to the first species.

Tempest et al., Bio/Technology 9, 266-271 (1991) discloses transferring CDRs from a murine monoclonal antibody to a human monoclonal antibody, then making additional alterations to one of the framework regions to restore binding affinity and specificity.

WO-A-9007861 discloses a humanized immunoglobulin having a human framework and CDRs from a second species. At least one framework amino acid is substituted by the corresponding amino acid from the donor Ig where the human amino acid is rare for that position but the donor amino acid in that position is common in humans.

### OBJECTS OF THE INVENTION

It is, accordingly, an objective of the present invention to provide a method of producing immunoglobulin according to claim 1.

### SUMMARY OF THE INVENTION

A unique method is disclosed for identifying and replacing immunoglobulin surface amino acid residues which converts the antigenicity of a first mammalian species to that of a second mammalian species. The method will simultaneously change immunogenicity and strictly preserve ligand binding properties. The judicious replacement of exterior amino acid residues has no effect on the ligand binding properties but greatly alters immunogenicity.

### DETAILED DESCRIPTION OF THE INVENTION

The present invention relates to a "humanization" procedure which simultaneously reduces the immunogenicity of the rodent monoclonal antibody while preserving its ligand binding properties in their entirety. Since the antigenicity of a protein is primarily dependent on the nature of its surface, the immunogenicity of an xenogenic or allogenic antibody could be reduced by replacing the exposed residues which differ from those usually found in another mammalian species antibodies. This judicious replacement of exterior residues should have little, or no, effect on the interior domains, or on the interdomain contacts. Thus, ligand binding properties should be unaffected as a consequence of alterations which are limited to the variable region framework residues. The process is refered to as "veneering" since only the outer surface or skin of the antibody is altered, the supporting residues remain undisturbed.

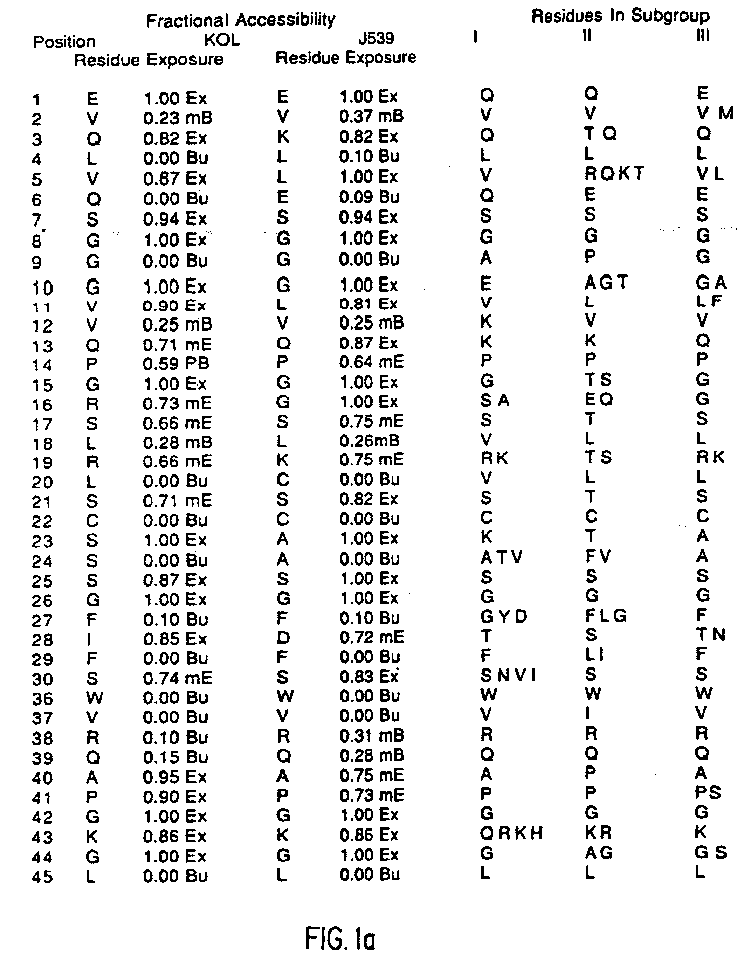

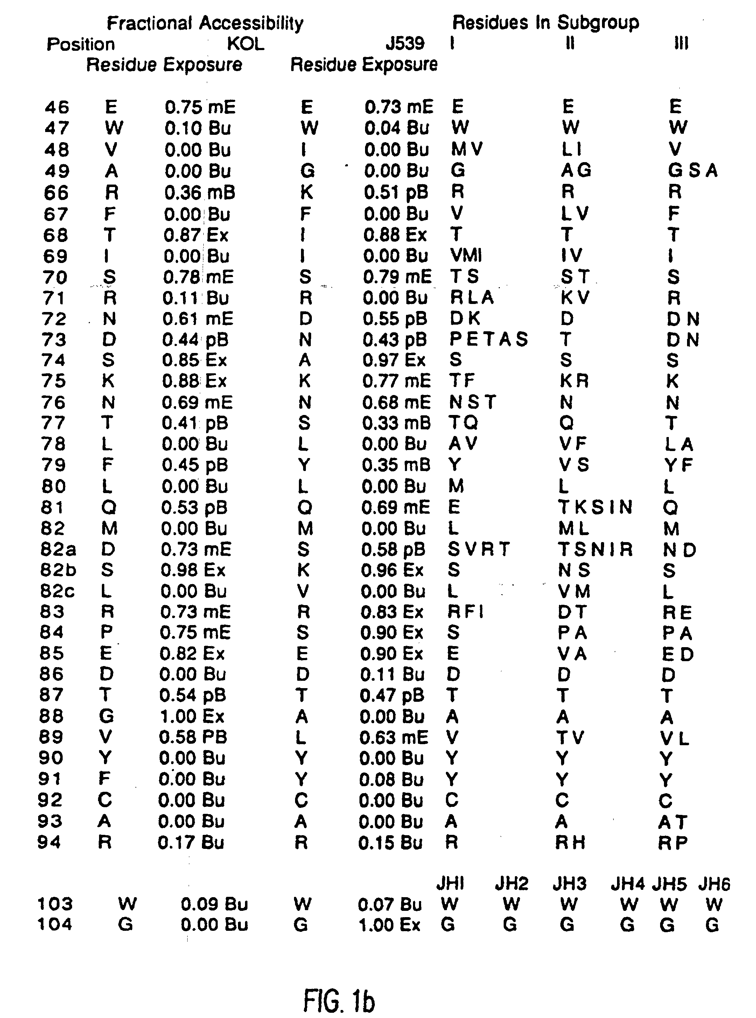

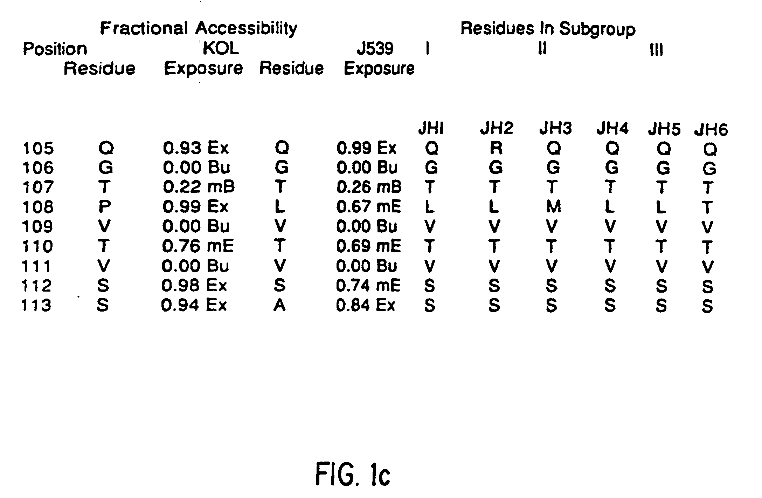

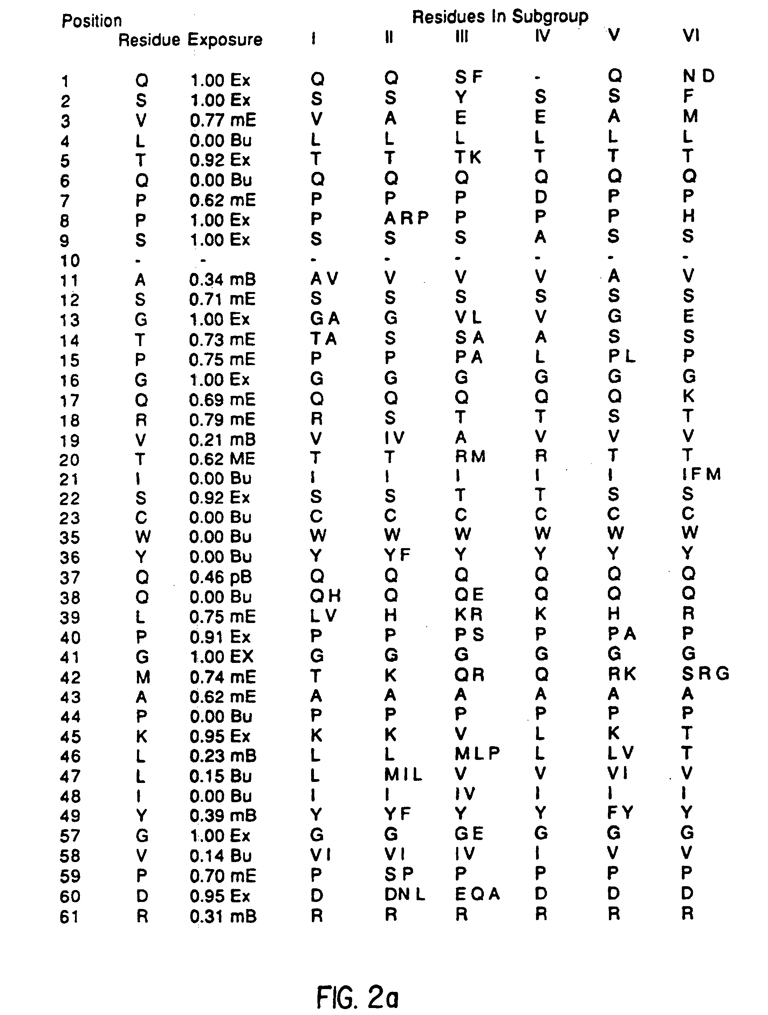

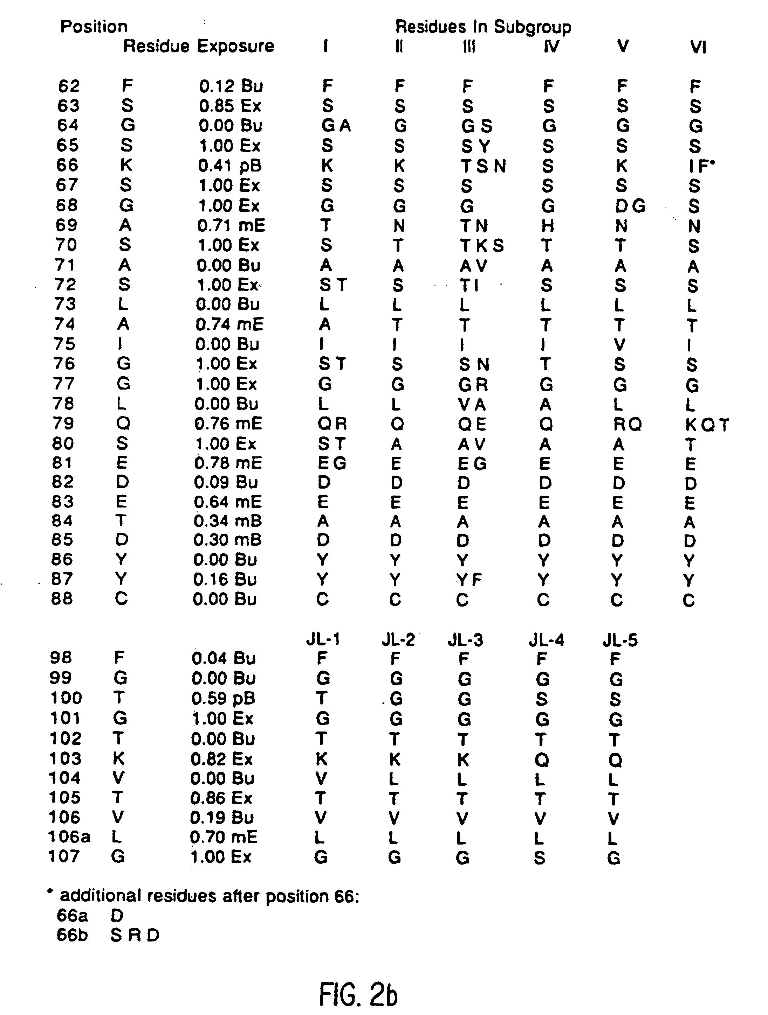

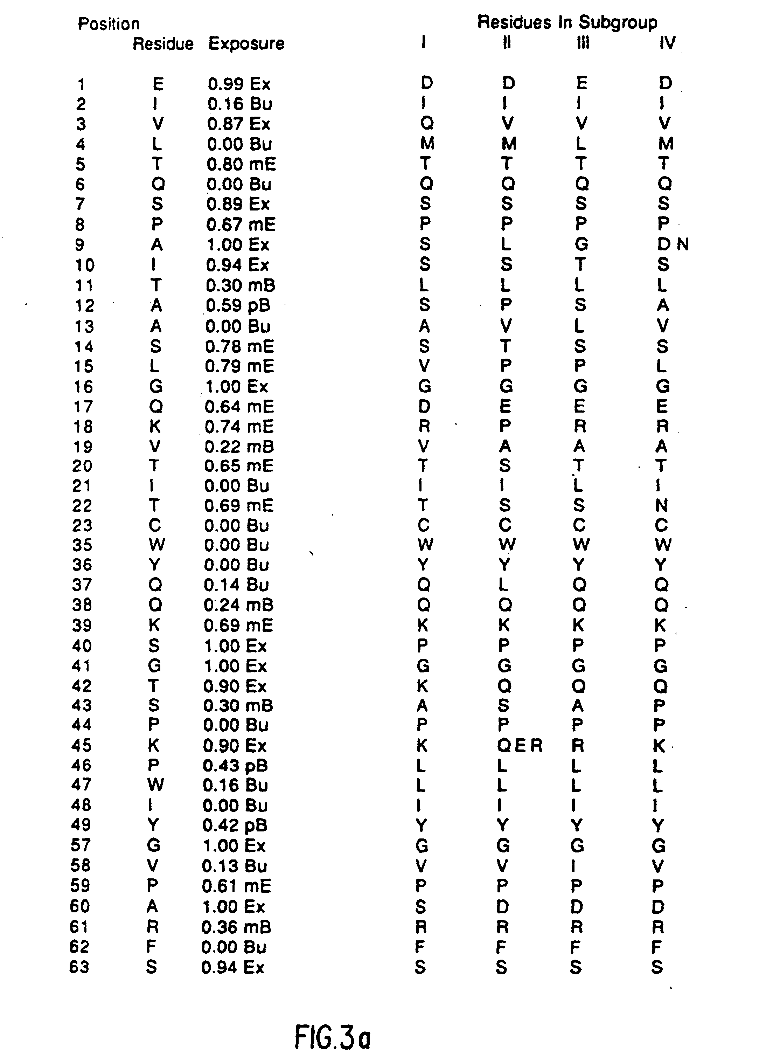

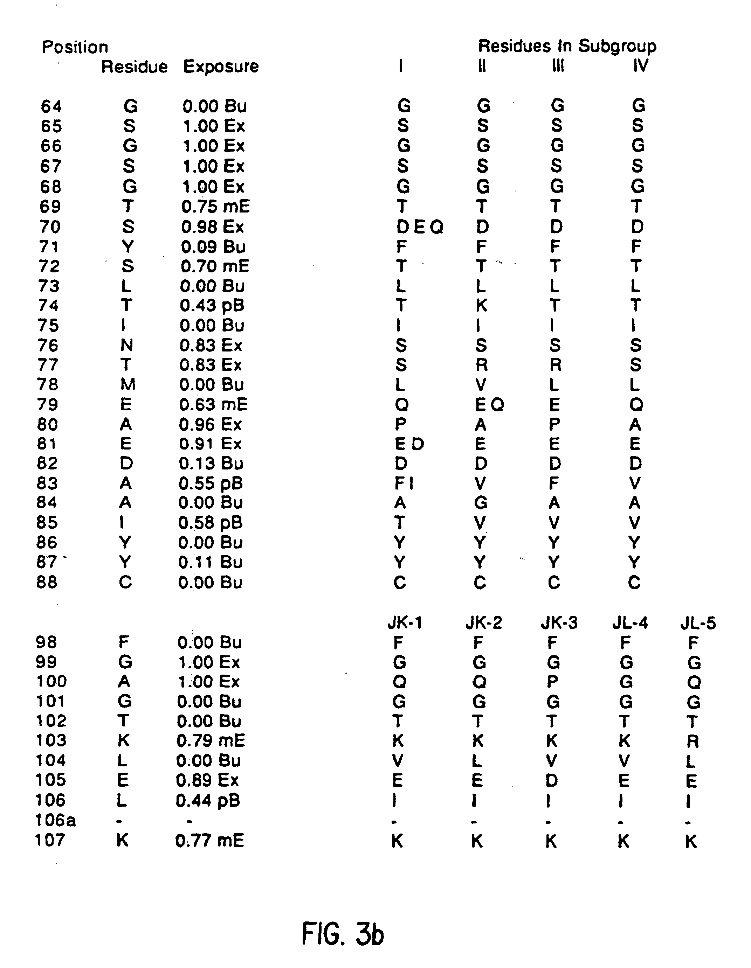

The procedure for "veneering" makes use of the available sequence data for human antibody variable domains complied by Kabat et al., "Sequences of Proteins of Immunological Interest", 4th ed., Bethesda, Maryland: National Institutes of Health, 1987, updates to this database, and other accessible U.S. and foreign databases (both nucleic acid and protein). The subgroups into which the various sequences have been combined are presented in Figures 1 - 3, indicating the most frequently occurring amino acid at each framework position. Also presented are the sequences of the various J-minigenes. The solvent accessibilities of the amino acids, as deduced from the known three-dimensional structure for human and mouse antibody fragments, are included in these figures.

High resolution X-ray crystallography of the variable domains of the antibodies KOL and J539 have been subjected to extensive refinement beginning with the structures available from the Protein Data Bank (Bernstein et al., J. Mol. Biol. 112: 535-542 1977; file 2FB4 for KOL and file 2FBJ for J529). The solvent accessibilities were computed as described by Padlan Proteins: Struct. Funct. Genet. 7: 112-124 (1990).

There are two steps in the process of "veneering". First, the framework of a first animal species, e.g. the mouse, variable domains are compared with those corresponding frameworks of a second animal species, e.g. human. It is intended that this invention will allow the antigenic alteration of any animal species antibody. The present invention illustrates the conversion of murine antibody to human antibody, but this is for illustrative purposes only. The most homologous human variable regions are then compared residue for residue to the corresponding murine regions. This will also define the human subgroup to which each mouse sequence most closely resembles. Second, those residues in the mouse framework which differ from its human counterpart are replaced by the residues present in the human counterpart. This switching occurs only with those residues which are at least partially exposed (mE and Ex; Figures 1-3) (i.e. those having a fractional accessibility of at least 0.61). One retains in the "veneered" mouse antibody: its CDRs, the residues neighboring the CDRs, those residues defined as buried or mostly buried (mB and Bu; Figures 1-3), and those residues believed to be involved with interdomain contacts (boldface, Figures 1-3).

Human and murine sequences frequently differ at the N-terminus of both heavy and light chains. The N-termini are contiguous with the CDR surface and are in position to be involved in ligand binding. Thus, wisdom would dictate that these murine termini be retained in its "veneered" version.

Finally, replacement of some amino acid types could have a significant effect on the tertiary structure or electrostatic interactions of the variable region domains. Hence, care should be exercised in the replacement of proline, glycine, and charged amino acids.

These criteria and the following procedures are used to prepare recombinant DNA sequences which incorporate the CDRs of a first mammalian species, animal, mMAb, both light and heavy chains, into a second mammalian species, human, appearing frameworks that can be used to transfect mammalian cells for the expression of recombinant human antibody with the antigen specificity of the animal monoclonal antibody. Preferrably the recombinant immunoglobulins will be recognized as self proteins when administered for threapeutic purposes. This method of "veneering" will render the recombinant antibodies useful as therapeutic agents because they will be either weakly immunogenic or non-immunogenic when administered to humans. The invention is further contemplated to include the recombinant conversion of any animal monoclonal antibody into a recombinant "human-appearing" monoclonal antibody providing that a suitable framework region can be identified (as described below). The animal monoclonals may include, but are not limited to, those murine monoclonal antibodies described by VanVoorhis et al., J. Exp. Med. 158: 126-145 (1983) which bind to human leukocytes and the appropriate mMAbs produced by hybridomas deposited in the Hybridoma Cell Bank maintained by the American Type Culture Collection (ATCC) and described in the ATCC Catalog of Cell Lines 8 Hybridomas, No. 6, 1988.

The CDR sequences from the animal monoclonal antibody are derived as follows. Total RNA is extracted from the murine hybridomas, for exampe the 1B4 myeloma cells described by Wright et al., Proc. Natl. Acad. Sci. USA 80: 5699-5703 (1983), the 60.3 cells described by Beatty et al., J. Immunol. 131:2913-2918 (1983), the TS1/18 cells described by Sanchez-Madrid et al., J. Exp. Med. 158: 1785-1803 (1983), and other anti-CD18 or CD11 monoclonal antibodies and hybridomas as described in Leukocyte Typing lll, Springer-Verlag, New York (1988), using standard methods involving cellular solubilization with guanidinium isothiocyanate (Chirgwin et al., Biochem. 18: 5294-5299 [1979]). The murine 1B4 mMAb will be used as the primary example of animal MAb that can be "veneered" by the unique process being disclosed. The invention is intended to include the conversion of any animal immunoglobulin to a "human-appearing" immunoglobulin. It is further intended that "human-appearing" immunoglobulin (Ig) can contain either kappa or lambda light chains or be one of any of the following heavy chain isotypes (alpha, delta, epsilon, gamma and mu).

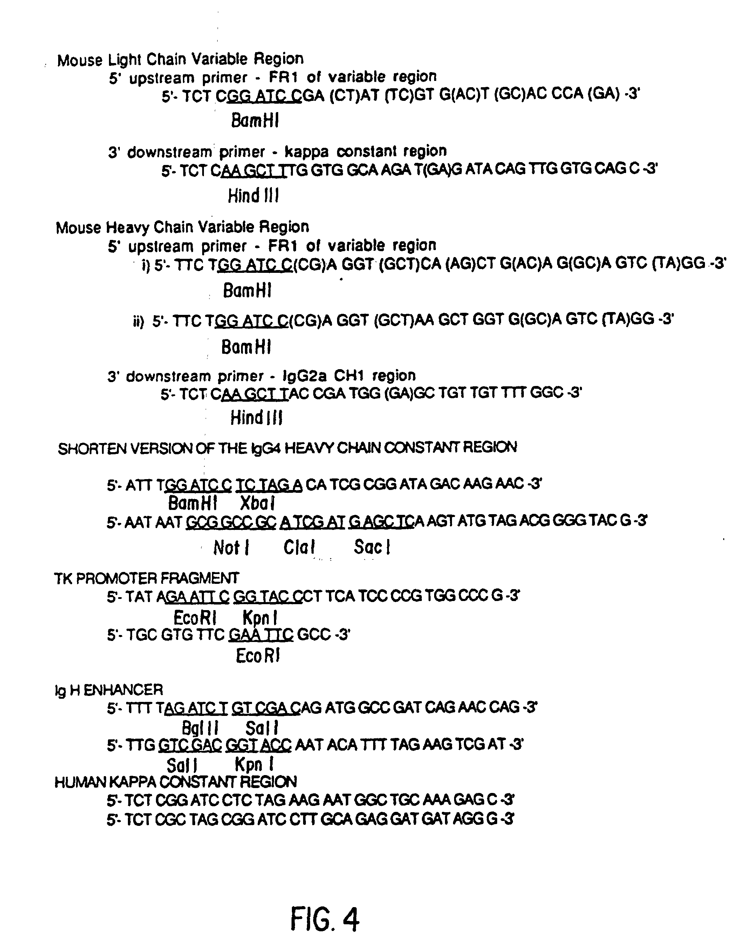

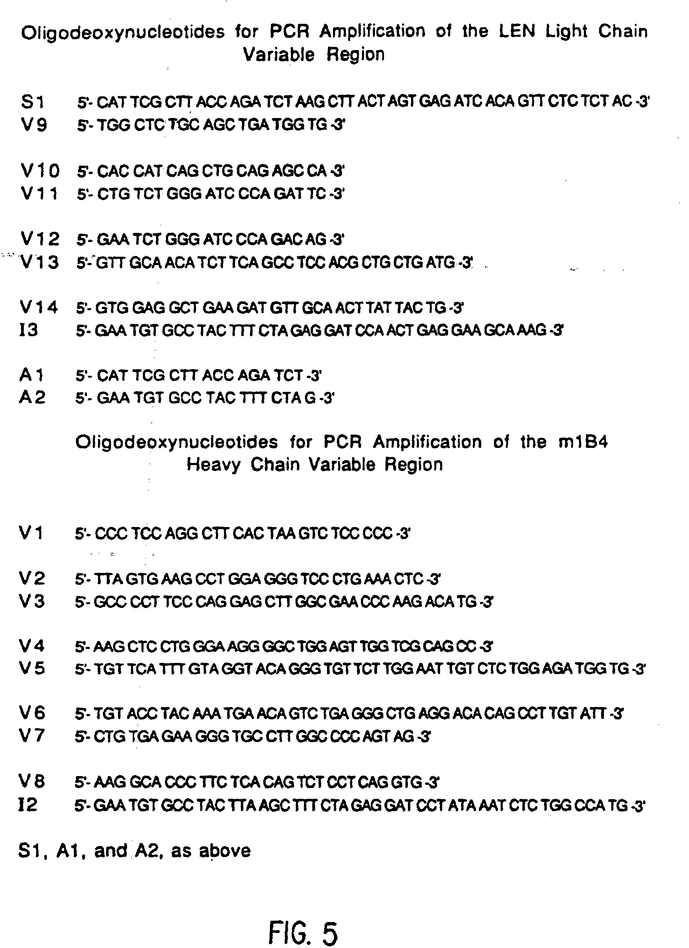

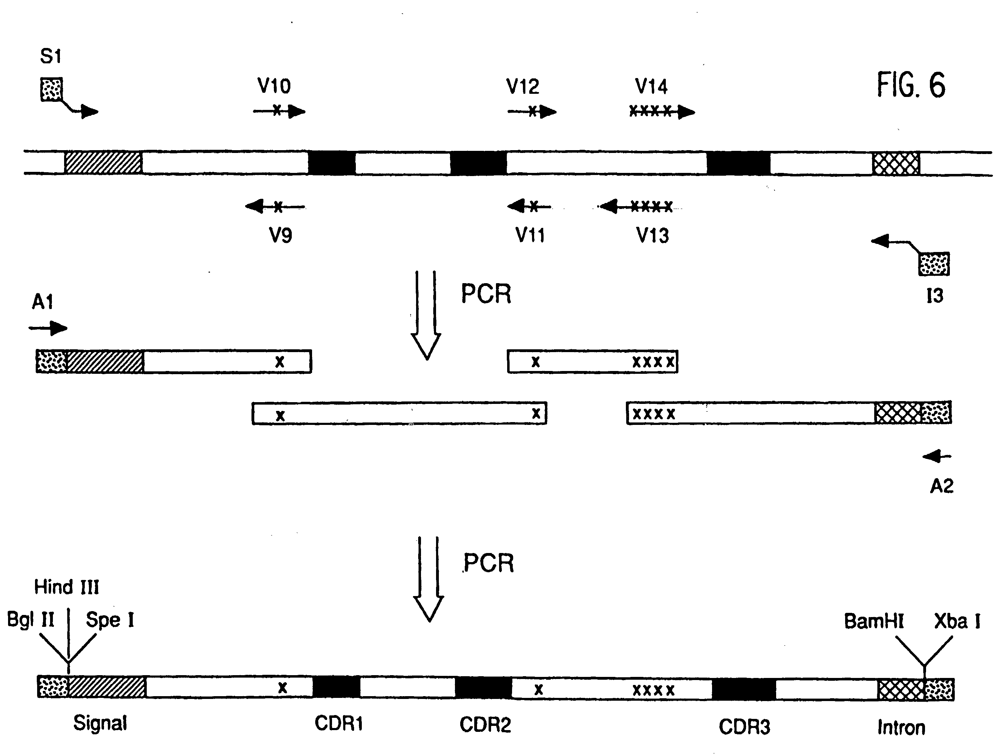

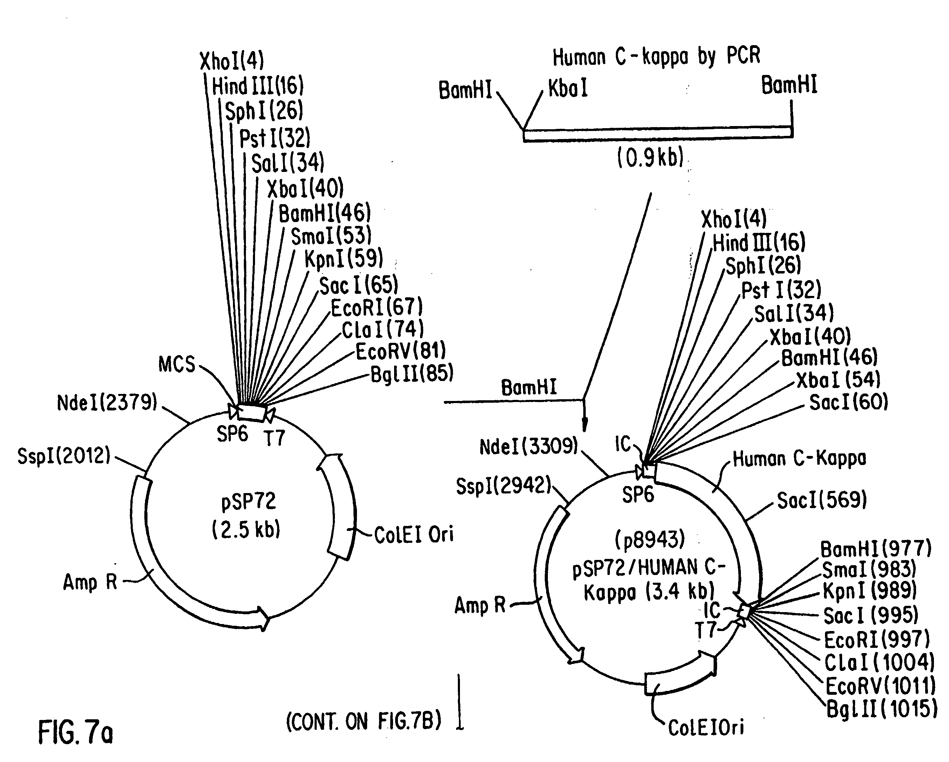

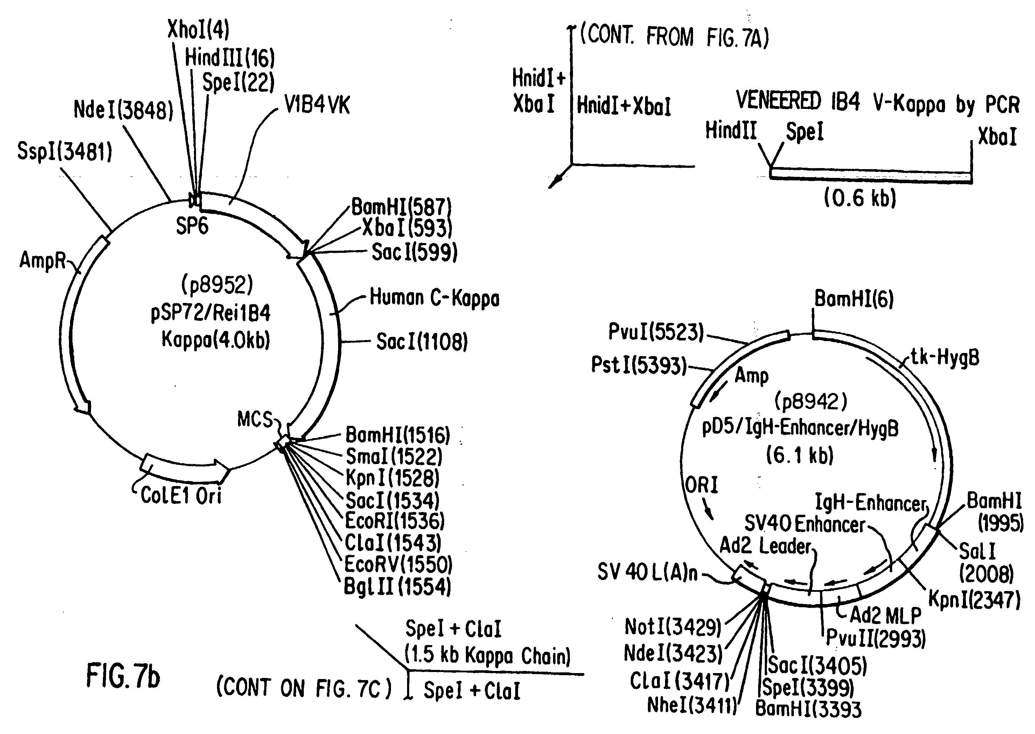

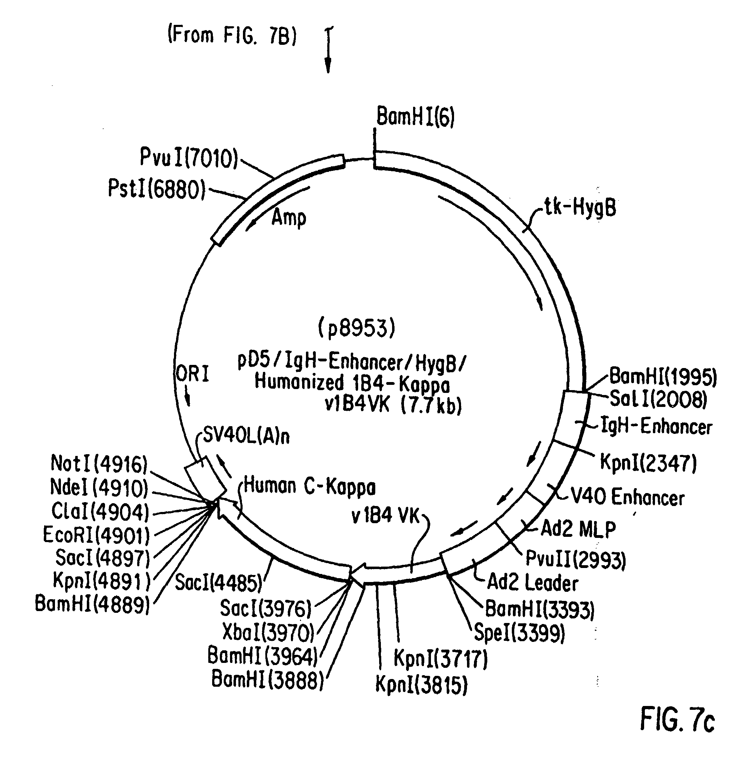

Pairs of degenerate oligodeoxynucleotide primers (Figure 4) representing sequences within framework 1 of the murine kappa light chain variable region and light chain constant domain, or those within framework 1 of the murine IgG2a heavy chain variable region and heavy chain constant CH1 domain are synthesized on an Applied Biosystem 381A DNA synthesizer, removed from the resin by treatment with concentrated NH₄0H and desalted on a NAP-5 column eluted with H₂0. Total RNA, about 2 µg, is reverse transcribed for 30 min at 42°C using Moloney MLV reverse transcriptase, about 200 units (BRL), and about 10 pmoles of the constant region complementary strand primers for either the heavy or light chain. The reverse transcriptase is heat inactivated, at about 95° C for about 5 min, and the reactions are made to contain in about 100 µl of PCR buffer about 50 pmoles of each of the paired primers and and 2.5 units of Taq polymerase. About 45 cycles of amplification (2', 94° C; 2', 55° C; 2' 72° C) are followed by gel purification of the anticipated 400+ base pair (bp) DNA fragments. Prior to subcloning those DNAs into a blunt-ended intermediate plasmid such as pSP72 (Promega) they are terminally phosphorylated using T4 polynucleotide kinase. Multiple clones representing these PCR amplified sequences are grown and submitted to DNA sequence determinations using Sequenase® and T7 and SP6 specific sequencing primers. A unique DNA sequence representing a murine IgG2a heavy chain variable region is obtained by analysis of the derived amino acid sequences. Replacement of the "murine-appearing" framework residues with those residues compatible with a human variable region is accomplished utilizing the following unique processes. An appropriate human framework is determined utilizing the criteria discussed below. The light chain variable region framework with sufficient homology to the the m1B4 framework was determined to be the human LEN framework (FR). The Len FR shows a similarity of 90% and an identity of 81% when compared to murine 1B4. This sequence, with its leader, 3' intronic sequences and engrafted m1B4 CDRs had been subcloned into the intermediate vector pGEM3Z (Promega), as described in Daugherty et al. Nucleic Acids Res. 19: (1991). About eight oligodeoxynucleotide primers (Figure 5) are synthesized representing the primers necessary to generate by polymerase chain reaction (PCR) amplification four DNA fragments. Incorporated into all but the terminal oligodeoxynucleotide primers were those sequences corresponding to the veneered MAb 1B4 light chain, with its unaltered CDRs, and at least 15 bases of 5'-terminal complementarity to allow for the subsequent PCR- directed recombination of these four fragments. For the purposes of exemplifying the "veneering" process the LEN light chain variable region already containing an engrafted set of CDRs representing those within the light chain of murine 1B4 was used as the template into which mutations were placed so as to easily create the "veneered" framework sequence. The appropriate primer pair (S1 & V9, V10 & V11, etc.), about 50 pmole each, was combined with about 10 ng of plasmid DNA representing the LEN CDR- grafted framework, about 2.5 units of Taq DNA polymerase and about twenty-five (25) cycles of PCR amplification ensued (cycle periods: 1', 94° C; 1', 55° C; 2' 72° C). The products of the four reactions, purified by agarose gel electrophoresis, are combined, about 10 ng of each DNA fragment, along with terminal oligodeoxynucleotide primers (A1 &A2, Figure 6) and Taq DNA polymerase. The combined fragments were PCR amplified (25 cycles of: 2', 94° C; 2', 55° C; 2' 72° C). Following restriction endonuclease digestion with Hind lll and Xba I the amplified DNA is purified by agarose gel electrophoresis and subcloned into compatible sites of an intermediate vector pSP72 (Promega) which contains the human kappa light chain constant region (see Figure 7). Genomic DNA, about 1 µg, purified from a human B cell line (GM0108A: NIGMS Human Genetic Mutant Cell Repository, Institute for Medical Research, Camden, NJ) is used as a template for PCR amplification (Figure 4) of about a 920 base pair fragment containing the splice acceptor for the kappa light chain constant domain, the exon and a portion of its 3'-untranslated region. The PCR product is purified by agarose gel electrophoresis, digested with Bam H1 endonuclease, and subcloned into pSP72 previously linearized with Bam H1. The individual clones representing the pSP72 intermediate vector containing both the 1B4 "veneered" light chain variable region and the human kappa constant region derived by PCR amplification of human DNA are used to determine the DNA sequence of the "veneered" light chain varisble region.

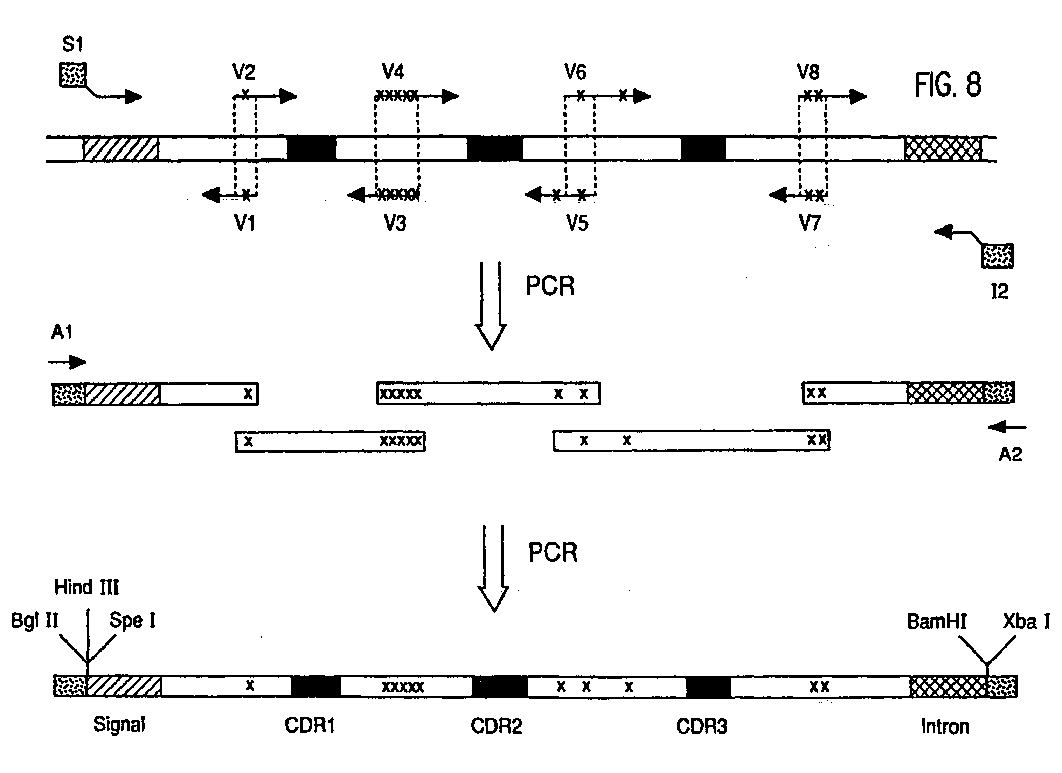

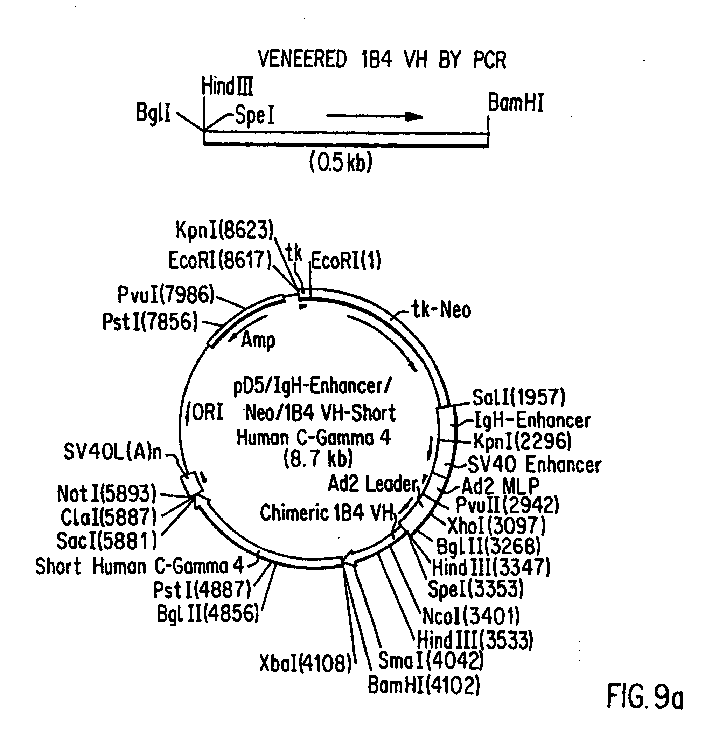

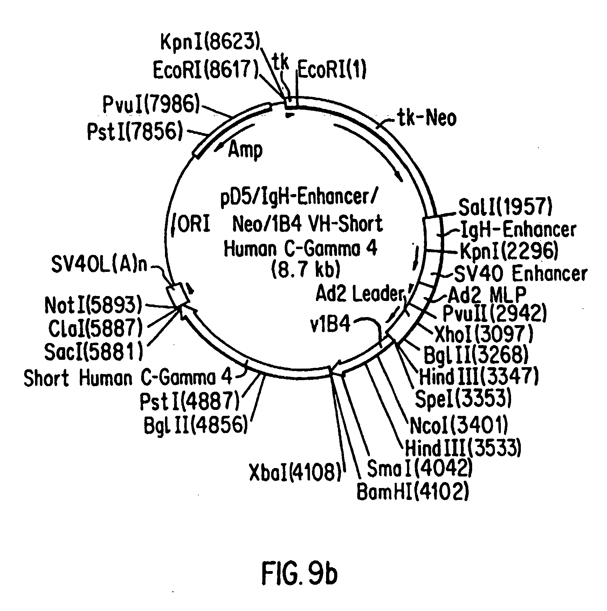

The "veneered" heavy chain portion of the recombinant antibody is derived from the mutated version of the murine 1B4 heavy chain variable region fused to the human constant region of a gamma 4 subtype obtained from a lambda library constructed by Flanagan and Rabbits, Nature 300: 709-713 (1982). The variable region of the "veneered" heavy chain is constructed from five DNA fragments representing a signal sequence, portions of the mutated murine heavy chain variable region, and an intronic sequence (Figure 8). Oligodeoxynucleotide primer pairs (Figure 5) are synthesized representing the primers necessary to generate by PCR amplification these five DNA fragments from about 10 ng of plasmid DNA template obtained from a pSP72 intermediate vector containing the heavy chain variable region previously used to determine the murine 1B4 CDR sequence. Amplification of the signal fragment, variable region fragments, and intron-containing fragment was as described above. The agarose gel purified products are combined, about 10 ng of each product, with terminal oligodeoxynucleotide primer pairs (Figure 8) and the PCR-generated in vitro recombined template is amplified using the standard procedures described above. Prior to subcloning into a Hind III and Bam HI digested expression vector containing the human heavy chain gamma 4 constant region (Figure 9), this recombined product is similarly digested and agarose gel purified. Individual clones are submitted to DNA sequence determination using Sequenase® and T7 and SP6 specific sequencing primers and one is chosen for subsequent expression. The gamma 4 heavy chain constant region is subcloned as about a 6.7 Kb Hind 111 fragment derived from the plasmid pAT84 into the Hind lll site of the intermediate vector pSP72. This plasmid is then used as the template DNA from which a shortened version of the gamma 4 constant region is subcloned using PCR amplification and the primer pairs indicated in Figure 4. Eukaryotic expression vectors are constructed as described below.

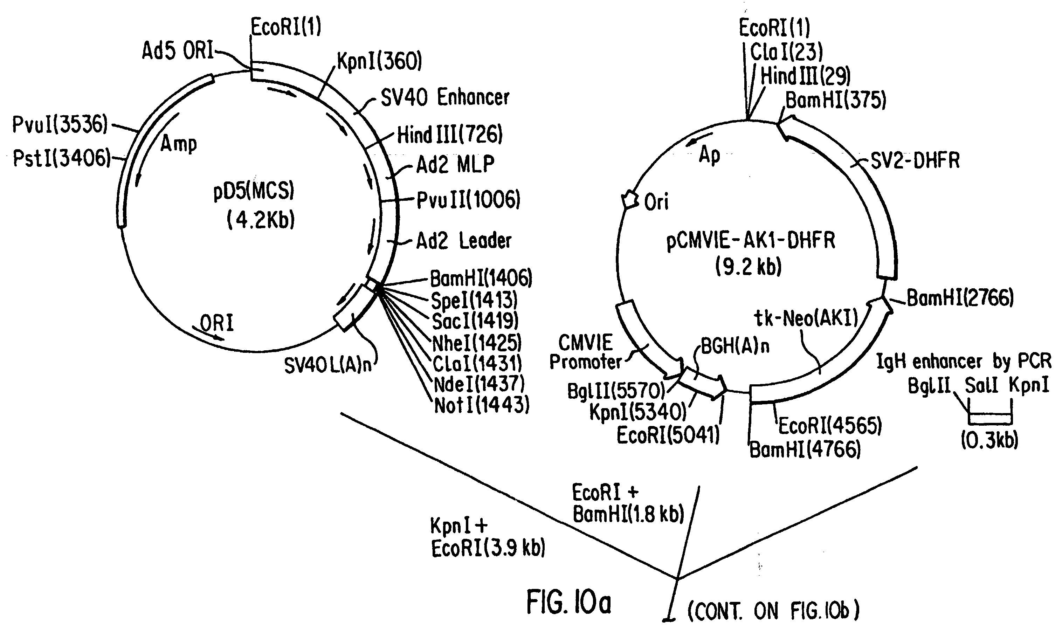

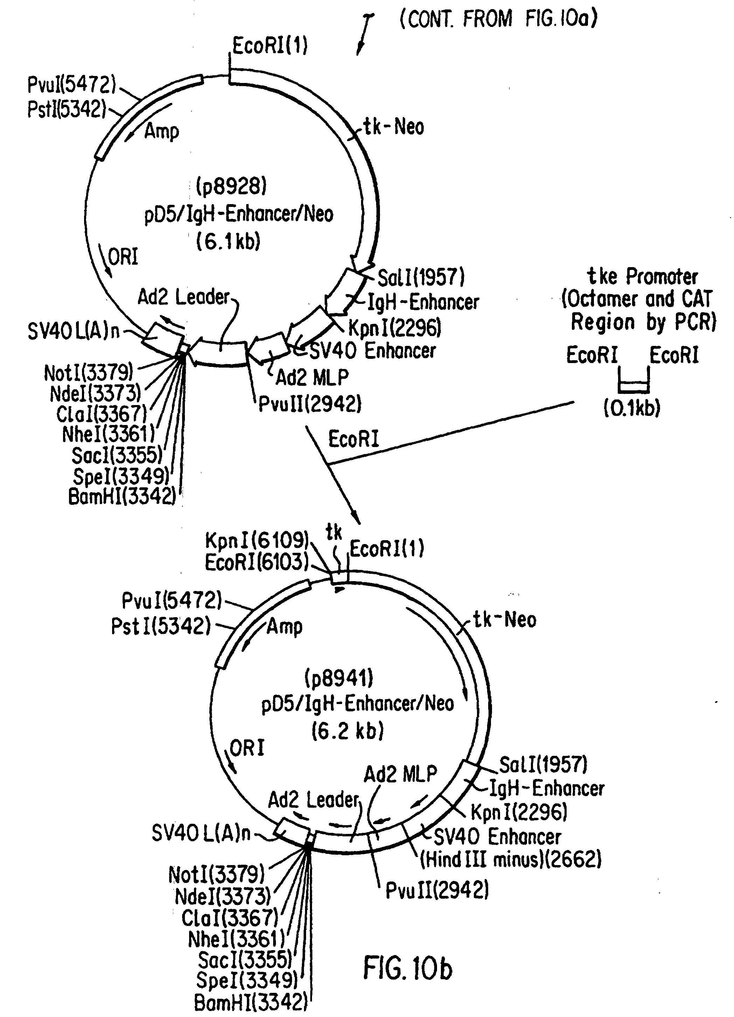

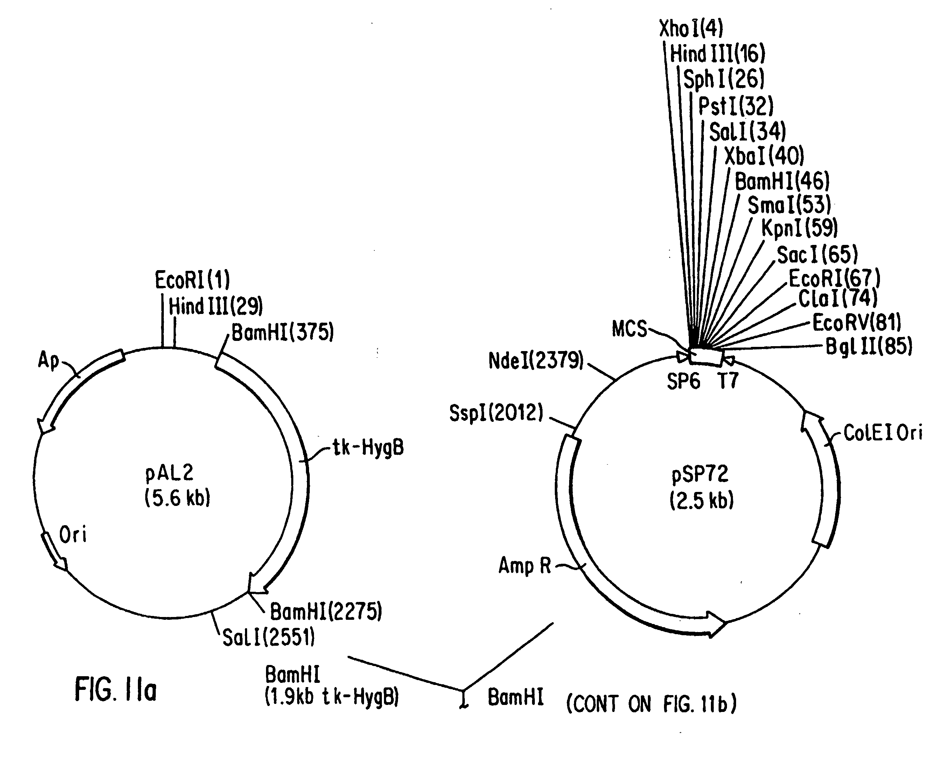

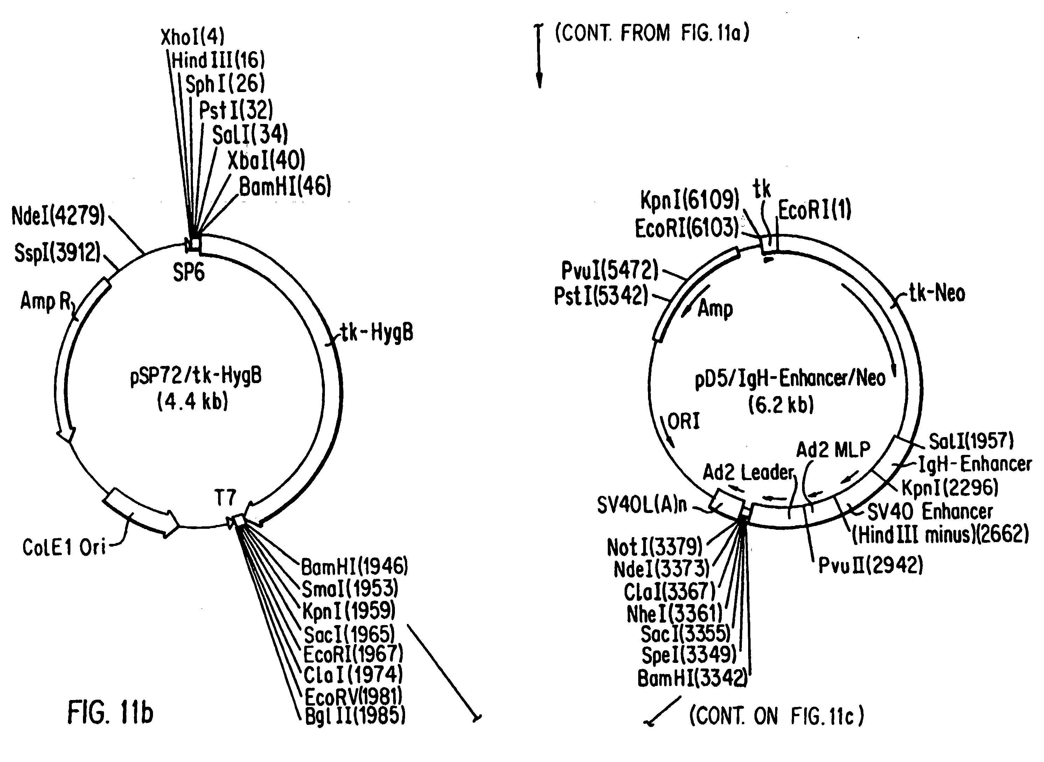

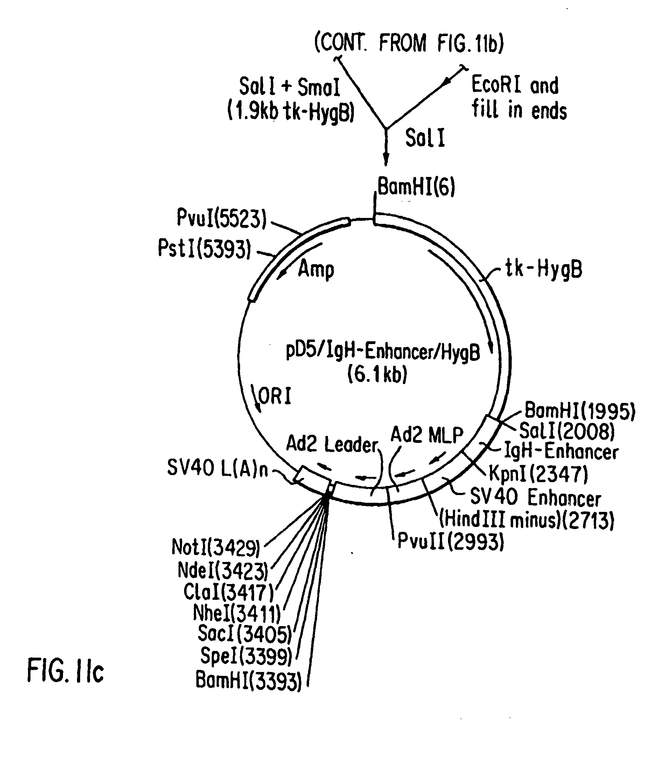

Expression vectors are defined herein as DNA sequences that are required for the transcription of cloned copies of genes and the translation of their mRNAs in an appropriate host. Such vectors can be used to express eukaryotic genes in a variety of hosts such as bacteria, blue-green algae, plant cells, yeast cells, insect cells and animal cells. The immunoglobulins may also be expressed in a number of virus systems. Specifically designed vectors allow the shuttling of DNA between host such as bacteria-yeast or bacteria-animal cells. An appropriately constructed expression vector should contain: an origin of replication for autonomous replication in host cells, selectable markers, a limited number of useful restriction enzyme sites, a potential for high copy number, and strong promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses. The heavy chain immunoglobulin molecule is transcribed from a plasmid carrying the neomycin (G418) resistance marker while the light chain immunoglobulin is transcribed from a plasmid carrying the hygromycin B resistance marker. With the exception of the drug resistance portion of these plasmids they are identical. The preferred progenitor of the immunoglobulin expression vectors is the pD5 (Berkner and Sharp, Nucl. Acids Res. 13: 841-857 [1985]) eukaryotic expression vector which contains the origin of adenovirus replication, the SV40 enhancer domain, the adenovirus major late promoter, the adenovirus 2 tripartite leader, a 5' splice donor from the adenovirus third leader and a 3' splice acceptor derived from an immunoglobulin locus, a multiple cloning site placed in the Bam H1 site subsequent to receipt of the vector, and the SV40 late polyadenylation signal (Figure 10). The origin of replication is removed by digestion with Eco R1 and Kpn I and replaced by two fragments representing the neo selectable marker gene (derived from plasmid pCMVIE-AK1-DHFR as an Eco R1/Bam H1 about1.8 Kb fragment) and the Ig heavy chain enhancer (obtained as a PCR amplified fragment using human DNA as the template, and the oligodeoxynudeotides listed in Figure 4 as the primer pair, following its digestion with Bgl ll and Kpn 1). The resultant expression vector is found to lack a small portion of the TK promoter responsible for the transcription of the neomycin gene. This is replaced by insertion into the Eco Rl site about a 0.14 Kb PCR amplified fragment derived from the CMVIE-AK1-DHFR DNA using the primer pair listed in Figure 4. The resultant heavy chain expression vector (p8941) is modified by removal of the indicated Hind lll and Xba I sites using standard procedures. To convert this vector into one expressing the hyg romycin B selectable marker the neomycin-resistance cassette is removed by digestion first with Eco R1 followed by DNA polymerase-directed fill in of the 5' overhand, then subsequent Sal I digestion. The about 1.9 Kb hygromycin B expression cassette, TK promoter and TK polyadenylation signal flanking the hygromycin B gene, (obtained as a 1.8 kb Bam H1 fragment in plasmid pL690, Gritz and Davies, Gene 25: 179-188 [1981]) is removed from the plasmid pAL-2 by Bam H1 digestion and subcloned into the Bam H1 site of the intermediate vector pSP72. The hygromycin B cassette is removed from this vector by digestion with Sma I and Sal I and cloned into the expression vector linearized as described above to create a blunt end and Sal I end DNA fragment (Figure 11).

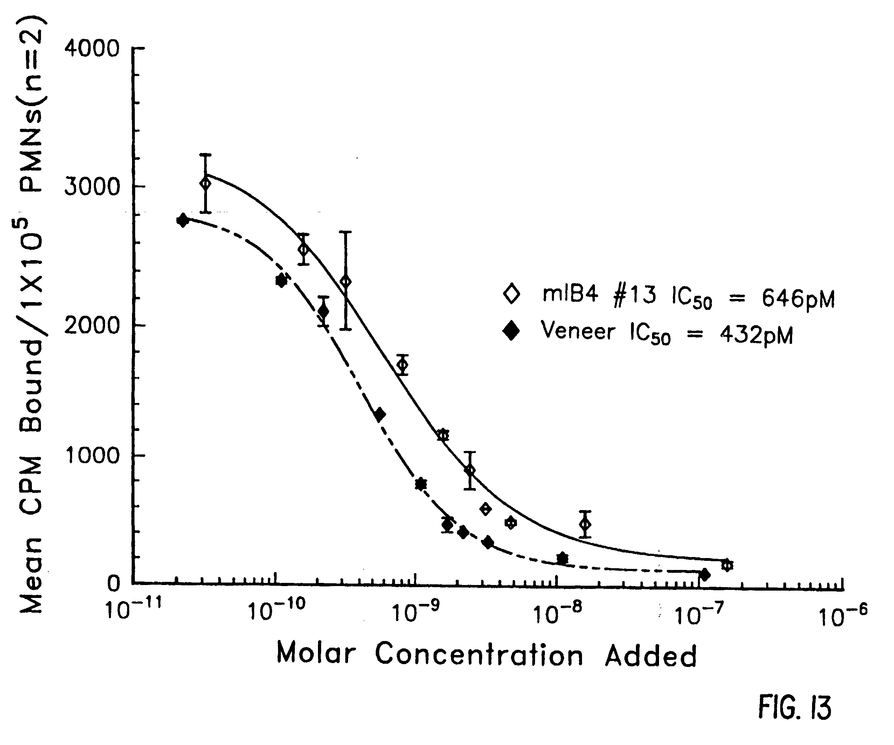

Expression of the 1B4 "veneered" kappa light chain is accomplished by transferring this cistron from the pSP72-based intermediate cloning vector (p8952), containing the human kappa constant region, to the hygromycin B selectable eukaryotic expression vector (Figure 7). An about 1.5 kb DNA fragment resulting from the endonuclease digestion of p8952 with Spe I and Cla I is purified by agarose gel electrophoresis and ligated into the expression vector which has previously been linearized, following digestion with the same two restriction enzymes; and agarose gel purified. The heavy chain eukaryotic expression vector is constructed in two steps. First, the p8950 vector containing the modified heavy chain variable region of murine 1B4 fragment is digested with Bgl ll and Bam H1. The agarose gel purified 0.75 kb fragment is ligated into the Bam H1 site of the p8941 vector and recombinant clones containing this fragment in the proper orientation are identified. Plasmid DNA from one such clone is linearized by Bam H1 digestion and ligated with a 1.78 Kb Bam H1 fragment representing a short version of the human gamma 4 constant region, derived from plasmid pAT84 by PCR amplification. Following the identification of clones containing these inserts in the appropriate orientation, plasmid DNAs (one which is referred to as p8953) are grown and purified for transfection into recipient mammalian cells. Equal amounts, about 10 µg, of the plasmids encoding the 1B4 "veneered" IgG4 heavy chain and the 1B4 "veneered" kappa light chain are transfected by standard calcium phosphate precipitation procedures into the monkey kidney cell line CV-1P or the human embryonic kidney cell line 293. The culture supernants, assayed by a trapping ELISA (described below), were found to contain a human kappa light chain / human IgG4 immuno-globulin. Immulon-2 (Dynatech Labs.) 96-well plates are coated overnight with about a 5 µg/ml solution of mouse anti-human kappa chain constant domain monoclonal antibody (cat. #MC009, The Binding Site, Inc., San Diego, CA) in about 0.1 M NaHCO₃ buffer (pH 8.2) at about 4° C, and blocked with about 1% bovine serum (BSA) in about 0.1 M NaHCO₃ for about 1 hour at about 25° C. After this and all subsequent steps, washing was performed with phosphate buffered saline (PBS). The wells are then challenged with conditioned medium containing recombinant anti-CD18 antibody, or with predetermined quantities of human IgG4/kappa purified by protein A Sepharose® (Pharmacia Fine Chemicals) chromatography from human IgG4 myeloma serum (cat. # BP026, The Binding Site, Inc.) All samples are diluted in PBS containing about 0.05% Tween®-20. About 100 µl aliquots are incubated for about 1 hour at about 37° C in triplicate, and standard calibration curves are constructed using IgG4 concentrations ranging from about 10 ng/ml to about 100 ng/ml. Bound and fully assembled human IgG4 (either native or the recombinant 1B4 human "veneered" IgG4 constructs) are detected with about 100 µl aliquots of a 1:500 dilution of mouse anti-human IgG4 Fc monoclonal antibody conjugated to alkaline phosphatase (cat #05-3822, Zymed Laboratories, Inc.) in phosphate buffered saline (PBS) containing about 1 % BSA. After incubation for about 1 hour at about 37°C and subsequent washing, the quantities of bound conjugate are detected by incubating all samples with a 1 mg/ml solution of p-nitrophenyl phosphate in 0.1 M 2,2' amino methyl-propanediol buffer, pH 10.3, for about 30 minutes at about 25° C. The adsorbance of the wells is determined with a UV Max ELISA plate reader (Molecular Devices) set at 405 nm. The antibody secreted by the transfected human 293 cells or monkey kidney CV1 P cells, either following transient expression or subsequent to stable clone isolation, is isolated by protein A chromatography, the concentration of recombinant human anti-CD18 antibodies determined by the trapping Elisa described above, and used to compete with the binding of radiolabeled murine 1B4 to the CD18 ligand on the surface of activated human PMNs. Affinities of r-anti-CD18 antibody constructs are determined using a competitive ¹²⁵I-1 B4 soluble binding assay with stimulated human polymorpho-nuclear leukocytes (PMNs). Purified murine anti-CD18 monoclonal antibody (50 ug) is iodinated using chloramine-T (Hunter, W.M. and Greenwood, F.C., Nature 194: 495-496, 1962), and the radiolabeled antibody purified using a Bio-Sil® TSK250 (Biorad, Richmond, CA) gel filtration HPLC column (which fractionates proteins in the range of 1-300 x 103 daltons) equilibrated in 0.1 M phosphate buffer, pH 7.0. Effluent radioactivity is monitored with an in-line detector (Beckman Model 170; Beckman, Fullerton,CA) and total protein measured at OD₂₈₀ with a Kratos Spectroflow 757 detector (Kratos, Mawah, N.J.). A single ¹²⁵l-1B4 peak composed of coincident OD₂₈₀ and radioactivity tracings characteristically elutes at about 6 minutes, 30 seconds following sample injection. Specific activity of the product is generally about 10 µCi/µg protein, and 97-99% of the counts are precipitable with 10% trichloroacetic acid. The binding of this radiolabeled antibody is assessed on human PMNs purified on a discontinuous Ficoll/Hypaque gradient (English, D. and Anderson, B.R., J. Immunol. Methods 5: 249-255, 1974) and activated with about 100 ng/ml phorbol myristate acetate for about 20 minutes at about 37°C (Lo et al., J. Exp. Med. 169: 1779-1793, 1989). To determine the avidity of antibodies for CD18 molecules on the PMN surface, about 1 x 10⁵ activated PMNs are incubated in a buffer such as Hanks balanced salt solution containing about 20 mM Hepes (pH 7.2), about 0.14 units aprotinin (Sigma Chemical Co.) and about 2% human serum albumin (binding buffer) containing about 1.3 ng ¹²⁵I-1B4 (2.8 x 10⁻¹¹ M) in the presence of increasing concentrations of unlabeled 1B4 antibody (about 10⁻⁷ to 10-¹⁵M) in about a 300 µl reaction volume for about 1 hour at about 4°C with constant agitation. Cell bound 1B4 is separated from the unbound antibody by centrifugation through a 0.5 M sucrose cushion ( 4,800 x g, 3 minutes); the tubes are frozen on dry ice, and the tips cut off and counted with an LKB gamma counter. The IC₅₀ of the anti-CD18 antibody for the inhibition of ¹²⁵I-1B4 antibody binding is calculated using a four parameter fitter program (Rodbard et al., In, "Radioimmunoassay and Related Procedures in Medicine", International Atomic Energy Agency, Vienna, vol 1,469 - 504, 1978). The affinity of the "veneered" r-anti-CD18 antibody for the CD18 ligand is determined in a similar manner using murine ¹²⁵I-1B4 antibody and increasing quantities, as determined by the trapping Elisa, of unlabeled r-anti-CD18. The results of the binding assays are shown in Figure 13 and indicate that the avidity of the "veneered" recombinant 1B4 antibody is equal to that of the murine 1B4 monoclonal antibody. This result shows that an antibody with presumptive human isotype may be recombinantly constructed from the murine parent antibody by the introduction of numerous point mutations in its framework residues and expressed fused to human kappa and gamma 4 constant domains without loss in avidity for the antigen. It can be inferred from this result that the point mutations within the framework regions do not alter the presentation of the murine 1B4 light chain and heavy chain CDRs. Many of the examples of construction of recombinant human antibodies containing complementary regions replaced by those found within murine monoclonal antibodies have resulted in loss of avidity for the ligand or antigen. Thus, although these latter transmutations are possible, the successful maintenance of avidity is not assured. This procedure described above demonstrates that when strict attention is paid to the framework regions, and the nature of the amino acids within each framework, "humanization" may potentially be achieved without the loss of avidity which accompanies the transfer of CDRs to the "generic" human frameworks ("humanization") employed by Winter, European Patent Publication No. 239,400, published September 30, 1987.

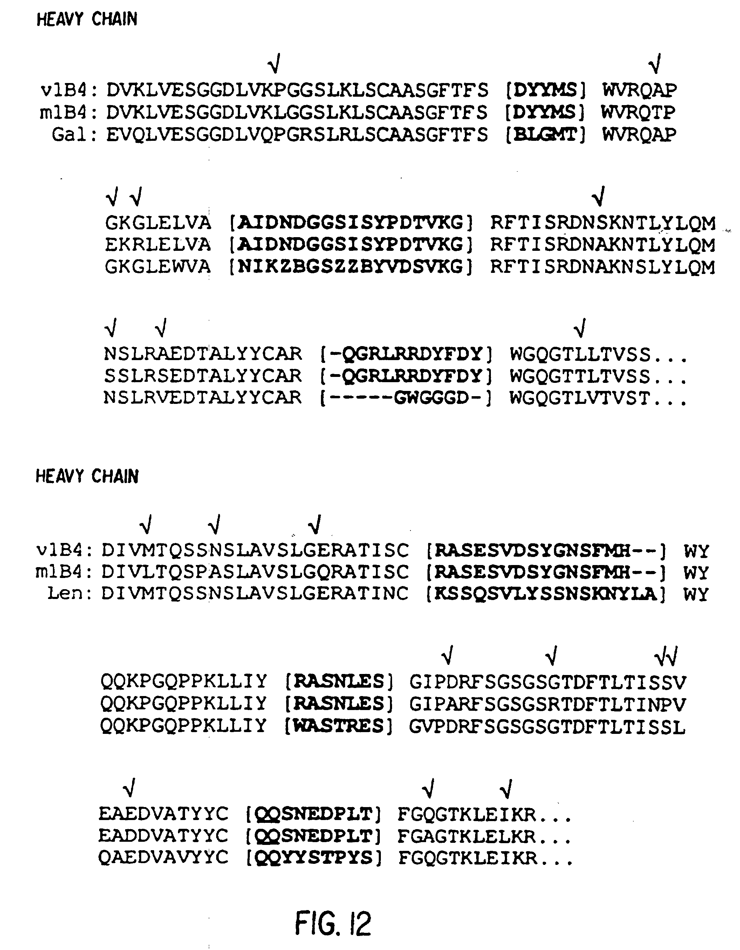

To identify human framework sequences compatible with the CDRs of, say, murine 1B4, human frameworks with a high degree of sequence similarity to those of murine 1B4 are identified. Sequence similarity is measured using identical residues as well as evolutionarily conservative amino acid substitutions. Similarity searches are performed using the murine 1B4 framework sequence from which the CDR sequences had been removed. This sequence is used to query a database of human immunoglobulin sequences that had been derived from multiple sources. Sequences with a high degree of sequence similarity are examined individually for their potential as humanizing framework sequences. In this way, the human homologue providing the murine CDRs with the structure most similar to their native murine framework is selected as the template for the construction of the "veneered" variable regions (Figure 12). Should human frameworks of sufficient similarity not be identifiable from compiled sequences, it is possible to isolate from human genomic DNA a group of closely related variable regions using recombinant technology. Thus, a degenerate 5' upstream oligodeoxynucleotide primer may be designed from the conserved sequences within the amino-terminus of each of the various human FR1 regions and paired with a degenerate 3' downstream oligodeoxynucleotide primers fashioned from the FR sequence determined from the murine monoclonal whose CDRs one wishes to transfer into a human context. These primer pairs are then used to PCR amplify from a human genomic template those DNA sequences which are flanked by the primer pair. The resulting DNAs may then be cloned and the DNA sequence derived from individual members will describe various murine-related human variable regions. The paucity of somatic mutations in framework residues and the conservation of amino acid sequence between mouse and man make this approach possible.

The construction of a complete recombinant human IgG4 antibody, whose heavy and light chain variable domains contain the CDR residues of the murine monoclonal antibody, with complete retention of the specificity and avidity of the parent murine monoclonal antibody is disclosed. The construction of the "veneered" light chain framework derived from the human sequence of LEN fused with a human kappa light chain constant region is described above. The murine variable region framework sequence, devoid of CDR sequences, is used to query a database of complete human variable region sequences. The human sequences that are most similar to the murine framework region are then analyzed individually to determine both their sequence identity and similarity to the murine framework region. In the case of murine 1B4 these sequences include, but are not limited to, "Gal", chosen because of its high degree of both similarity and identity with the 1B4 heavy chain sequence. The Gal FR has been found to be 85% similar and 79% identical to murine 1B4. These values are based upon the Dayhoff similarity matrix of evolutionarily conserved amino acid substitutions (R. M. Schwartz, M. O. Dayhoff, in Atlas of Protein sequence and structure M. O. Dayhoff, Eds. (National Biomedical Research Foundation, Washington, DC [1979]) (Figure 12). To prepare a recombinant DNA encoding the murine heavy chain CDRs in the context of a human-appearing framework the following procedures are performed. A set of ten short oligodeoxynucleotides are synthesized. Each pair is combined in a separate PCR reaction with the DNA template representing the murine 1B4 heavy chain variable region, amplified and isolated following PCR of the RNA of the murine hybridoma 1B4 as described above. Thus, about 50 pmole of each primer pair was combined with about 10 ng of plasmid DNA representing the murine 1B4 heavy chain variable region, about 2.5 units of Taq DNA polymerase and about twenty- five (25) cycles of PCR amplification ensued (cycle periods: 1', 94°C; 1', 55°C; 2' 72°C). The products of the five reactions (Figure 8) encoded portions of the 1B4 heavy chain variable region, beginning with the signal peptide encoding region and ending with the 3' intronic sequence which resides between the variable region coding domain and the IgG4 constant region sequence, with the desired point mutations to create a "veneered" variable region framework. These five fragments are purified by agarose gel electrophoresis, combined, about 10 ng of each DNA fragment, along with terminal oligodeoxynucleotide primers (A1 &A2, Figure 5) and Taq DNA polymerase. The combined fragments were PCR amplified (25 cycles of: 2', 94°C; 2', 55°C; 2' 72°C). By virtue of the complementary ends of the five fragments, the polymerization/denaturation/polymerization cycles of the polymerase chain reaction result in the formation, and subsequent amplification, of the combined sequences. Following 25 cycles of amplification the combined 0.8 Kb fragment is electrophoretically purified from an agarose gel and was digested with restriction enzymes Spe I and Bam H1. Following agarose gel electrophoresis, the purified DNA fragment is ligated into the heavy chain expression vector, p8958 (see Figure 9), in place of the chimaeric variable region existing in this vector. Each "veneered" variable region, with its associated human constant region, residing within a pD5-based expression vector plasmid was co-transfected into 293 cells and CV1 P cells and recombinant human antibody is found to be present in the conditioned medium 48 hours post transfection. The "veneered" recombinant antibody is isolated by protein A chromatography. The avidity of this antibody for the CD18 ligand displayed on the surface of activated human PMNs is compared with that of the murine 1B4 monoclonal antibody parent. Figure 13 shows that although each antibody contains the same set of six CDRs within different framework domains, they exhibit identical avidity for the ligand. Thus, the avidity of an antibody molecule does not rely upon the variable region framework residues which are surface exposed, rather the proper structure in which the CDRs are presented must be significantly influenced by the buried and inter/intra active residues. The parent murine monoclonal antibody demonstrates an IC₅₀ of about 1.0 to about 0.7 nM, the "veneered" molecule has a similar IC₅₀.

Further a method of inhibiting the influx or migration of leukocytes capable of expressing CD18 antigen (leukocyte integrin, beta subunit) on their surface into a site of inflammation or a tissue area or organ that will become inflamed following an influx of the cells is disclosed. The inflammation may result from an infection with pathogenic microorganisms such as gram-positive and gram-negative bacteria, parasites and fungi. The response may also be induced by viruses and non-infectious means such as trauma or reperfusion following myocardial infarction or stroke, immune responses to foreign antigen and autoimmune responses. The recombinant human anti-CD18 antibodies are useful in the treatment of inflammation in lung, central nervous system, kidney, joints, endocardium, eyes, ears, skin, gastrointestinal tract and urogenital system. Disease states in which the recombinant human anti-CD18 antibodies are useful as therapeutic agents include, but are not limited to: infectious diseases where active infection exists at any body site, such as meningitis; conditions such as chronic or acute secondary inflammations caused by antigen deposition; and other conditions such as, encephalitis; arthritis; uveitis; colitis; glomerulonephritis; dermatitis; psoriasis; and respiratory distress syndrome associated with sepsis and/or trama. Other inflammatory diseases which may be responsive to recombinant human anti-CD18 antibody include, but are not limited to, immune disorders and conditions involving T-cell and/or macrophage attachment/recognition, such as acute and delayed hypersensitivity, graft vs. host disease; primary autoimmune conditions such as pernicious anemia; infection related autoimmune conditions such as Type I diabetes mellitis; flares during rheumatoid arthritis; diseases that involve leukocyte diapedesis, such as multiple sclerosis; antigen-antibody complex mediated diseases including certain of the secondary infection states listed above; immunosuppression; and transplant rejection. Inflammatory conditions due to toxic shock or trauma such as adult respiratory distress syndrome and reperfusion injury; and disease states due to leukocyte dyscrasias and metastasis, are foreseen. Inhibition of leukocyte-endothelial attachment for diagnostic and therapeutic purposes; such as the iatrogenic opening of the endothelium to prevent the ingress of leukocytes during the ingress of a therapeutic drug in the instance of chemotherapy; or to enhance the harvesting of leukocytes from patients is also anticipated.

Recombinant human anti-CD18 antibodies or an active fragment thereof can be used to treat the above mentioned diseases. An active fragment will include the F(ab')2, the Fab and any other fragment that can bind to the CD18 antigen. Recombinant human anti-CD18 antibodies can be administered alone for non-infectious disease states or combined with antibiotics or other anti-infective agents for the treatment of infectious diseases for reasons discussed above. Administration will generally include the antibodies and possibly other substances in a physiologically acceptable medium or pharmaceutical carrier. Such physiologically acceptable media or phamaceutical carriers include, but are not limited to, physiological saline, phosphate buffered saline, phosphate buffered saline glucose, buffered saline and the like. The antibodies and any anti-infective agent will be administered by parenteral routes which include intravenous, intramuscular, subcutaneous and intraperitoneal injection or delivery. The amount of the antibodies and the mixture in the dosage form is dependent upon the particular disease state being treated. The amount of the recombinant human anti-CD18 antibody utilized in a dosage form can range from about 1 to about 1,000 mg, with a range of from about 10 mg to about 100 mg being preferred. The antibodies can be administered daily or less than daily as determined by the treating physician. The following examples illustrate the present invention without, however, limiting the same thereto.

### EXAMPLE

### Preparation of a "Veneered" Recombinant Antibody

An antibody was produced in which the variable domain of the light chain comprises the framework region of a murine light chain modified to contain surface exposed amino acids of human derivation. The variable domain of the heavy chain is similarly derived from the murine heavy chain with point mutations which replace murine exposed residues with human-appearing residues. The light chain human framework region was derived from human myeloma protein LEN. The CDR and framework sequences from the murine monoclonal antibody 1B4 which binds to CD18 (the beta subunit of the leukocyte integrin B-2 family which includes: LFA-1, Mac-1, and p150.95) were derived as follows. The hybridoma designated 1B4 which produces 1B4 monoclonal antibody was deposited under the Budapest Treaty at the International Depository Authority: American Type Culture Collection, 12301 Parklawn Drive, Rockville, MD, 20852. Viability was determined on June 6, 1989 and the hybridoma was designated HB 10164. Previous experiments had determined this antibody to be an IgG 2a with a kappa light chain (Wright et al., Proc. Natl. Acal. Sci. USA 80: 5699-5703 [1983]).

Total RNA was extracted from the 1B4 myeloma cells using standard methods involving cellular solubilization with guanidinium isothiocyanate (Chirgwin et al., Biochem. 18:5294-5299 [1979]). Sets of degenerate oligodeoxynucleotide primers (Figure 4) representing sequences within framework 1 of the murine kappa light chain variable region and kappa light chain constant domain, or those within framework 1 of the murine IgG2a heavy chain variable region and heavy chain constant CH1 domain were synthesized by standard phosphoramidite procedures on an Applied Biosystem 381A DNA synthesizer. Removal of the oligodeoxy-nucleotides (oligos) from the resin was accomplished by treatment with concentrated NH₄OH followed by desalting on a NAP-5 column (Pharmacia) with H₂O elution (when the oligos were <45 bases in length), or by use of an OPC column (Applied Biosystems Inc) with 20% acetonitrile elution (when the oligos were >45 bases in length), as recommended by the manufacturers. Total RNA (2µg) was reversed transcribed for 30' at 42°C using Moloney MLV reverse transcriptase (200 units, BRL) and 10 pmoles of the constant region complementary strand primers representing either heavy or light chain in a buffer (final volume of 20 µl) containing 50 mM Tris HCl, pH 8.3, 75 mM KCl, 3 mM MgCl₂, 10 mM DTT, and 20 units of RNAsin (Pharmacia). The reverse transcriptase was heat inactivated (95°C, 5') and the reactions were made to contain in 100µl of PCR buffer (10 mM Tris HCI, pH 8.3, 50 mM KCI, 1.5 mM MgCl₂, 0.01% gelatin, 200 µM each dNTP), 50 pmoles of each of the paired primers, and 2.5 units of Taq polymerase (Perkin Elmer/Cetus). Polymerase chain reaction (PCR) amplification was carried out essentially as described by Saiki et al., Science 230: 1350-1354 (1985) and others (Mullis et al., Cold Srping Harbor Symp. Quant. Biol.51: 263-273 [1986], Dawasaki and Wang, PCR Technology, Princples and Applications for DNA Amplification, Erlich, Ed., Stockton Press, NY, pp. 89-97 [1989], Tung et al., ibid. pp. 99-104 [1989]). Forty five cycles of amplification by a DNA Thermal Cycler (Perkin Elmer Cetus Instruments)(2', 94°C; 2', 55°C; 2' 72°C) were followed by gel purification of the anticipated 400+ base pair (bp) DNA fragments. Prior to subcloning the DNAs into a blunt-ended intermediate plasmid (pSP72, Promega) they were terminally phosphorylated using T4 polynucleotide kinase (Boehringer Mannheim). Multiple clones representing these PCR amplified sequences were isolated form DH5 transformed E.coli plated on LB agar plates containing 50 µg/ml ampicillin, grown by described procedures (Maniatis et al., Molecular Cloning, A Laboratory Manual, Cold Spring Harbor Laboratory, Cold Spring Harbor, NY, 1982), plasmid DNAs were extracted from the bacteria using the DNA preparation procedures of Birnboin and Doly, Nucleic Acid Res. 7: 1515 (1979), and the double-stranded plasmid DNAs were submitted to DNA sequence determinations using Sequenase® (United States Biochemicals) and T7 and SP6 specific sequencing primers (Boehringer Mannheim) using the protocols recommended by the manufacturer. A unique DNA sequence representing a murine IgG2a heavy chain variable region was obtained, as was a kappa light chain variable region sequence.

To give the final appearance of a "veneered" murine light chain, several residues within a template composed of the human LEN framework, into which had been grafted the CDRs described for 1B4, were replaced by corresponding residues found in the murine 1B4 light chain framework. Replacement of the human LEN variable region residues with those unique to MAb 1B4 took place as follows. Eight oligodeoxynucleotides (Figure 5) were synthesized representing the primers necessary to generate by PCR amplification four DNA fragments. Incorporated into all but the terminal oligodeoxynucleotides were those sequences corresponding to the MAb 1B4 light chain variable region framework residues to be point mutated and at least 15 bases of 5'-terminal complementarity (see Figure 6). The appropriate primer pair (50 pmole each) was combined with 10 ng of a 1B4 CDR-grafted LEN framework-containing plasmid DNA, 2.5 units of Taq DNA polymerase, PCR reaction components and buffer, and twenty-five (25) cycles of PCR amplification ensued (cycle periods: 1', 94°C; 1', 55°C; 2' 72°C). The products of the four reactions, purified by agarose gel electrophoresis, were combined (10 ng of each DNA fragment) along with a terminal oligodeoxynucleotide primer pair (amplifier) (Figures 5 & 6), Taq DNA polymerase, PCR reaction components and buffer, and the subsequent recombined fragments were amplified, as described above, for twenty-five (see Figure 6). Following restriction endonuclease digestion with HindIII and XbaI the amplified DNA was purified from an agarose gel and subcloned into these same sites of an intermediate vector pSP72 (Promega) which contained the human kappa light chain constant region, obtained as follows. DNA (1µg) purified from a human B cell line (GM01018A; NIGMS Human Genetic Mutant Cell Repository, Institute for Medical Research, Camden, N.J. 08103) was used as a template for the oligodeoxynucleotide primers described in Figure 4 to PCR amplify a 920 base pair fragment containing the splice acceptor for the human kappa light chain constant domain, the exon and a portion of its 3'-untranslated region (PCR primer pair choice was selected based on the kappa constant region sequence described by Hieter et al., Cell 22: 197-207 (1980). The PCR product was purified by agarose gel electrophoresis, digested with Bam H1 endonuclease, and subcloned into pSP72 (Promega) previously linearized with Bam H1.

The individual clones representing the pSP72 intermediate vector containing both the 1B4 "veneered" light chain variable region derived as described above, and the human kappa constant region, derived by PCR amplification of human DNA, were used to verify the DNA sequence of the "veneered" light chain variable region. The "veneered" heavy chain portion of the recombinant antibody was derived from a point mutated murine 1B4 heavy chain variable region fused to the human constant region of gamma 4 subtype obtained from a lambda library constructed by Flanagan and Rabbitts, Nature 300: 709-713 (1982).

The variable region of the "veneered" heavy chain was constructed from five DNA fragments representing a signal sequence, mutated portions of the murine 1B4 heavy chain variable region, and an intronic sequence (Figure 8). Oligodeoxy-nucleotide primer pairs (Figure 5) were synthesized representing the primers necessary to generate by PCR amplification these five DNA fragments from 10 ng of plasmid DNA template containing the murine 1B4 heavy chain variable region previously used to determine the murine 1B4 CDR and framework sequences. Amplification of the five fragments was performed as described above for the four light chain variable region fragments. The agarose gel purified products were combined (10 ng of each product) with terminal primer pairs (Figure 5) and the PCR-generated *in vitro* recombined template was amplified using the standard procedure also described above for recombining the fragments comprising the "veneered" light chain variable region. Prior to subcloning into a Hind III and Bam HI digested expression vector this recombined product was similarly digested and agarose gel purified. DNA was obtained following growth of individual bacterial clones and submitted to DNA sequence determination using Sequenase® and T7 and SP6 specific sequencing primers in order to verify the sequence of the reconstructed variable region and its flanking domains.

The gamma 4 heavy chain constant region had been subcloned as a 6.7 Kb Hind III fragment derived from the plasmid pAT84 (Flanagan and Rabbitts, supra) into the Hind III site of the intermediate vector pSP72 (Promega). This plasmid was then used as the template DNA from which a shortened version of the gamma 4 constant region was obtained using the standard PCR amplification procedures described above and the primer pairs indicated in Figure 4. Eukaryotic expression vectors were constructed as described below such that the heavy chain immunoglobulin molecule was transcribed from a plasmid carrying the neomycin (G418) (Rothstein and Reznikoff, Cell 23: 191-199 [1981]) resistance marker, while the light chain immunoglobulin was transcribed from a plasmid carrying the hygromycin B resistance marker (Gritz and Davies, Gene 25: 179-188 [1983]). With the exception of the drug resistance portion of these plasmids they are identical.

The progenitor of the immunoglobulin expression vectors was the pD5 eukaryotic expression vector (Berkner and Sharp, Nucl. Acids Res. 13: 841-857 [1985]) which contained the origin of adenovirus replication, the SV40 enhancer domain, the adenovirus major late promoter, the adenovirus 2 tripartite leader, a 5' splice donor from the adenovirus third leader and a 3' splice acceptor derived from an immunoglobulin locus, a multiple cloning site, and the SV40 late polyadenylation signal (Figure 10). The origin of replication was removed by digestion with Eco R1 and Kpn I and replaced by two fragments representing the neo selectable marker gene (derived from plasmid pCMVIE-AK1-DHFR (Silberklang et al., Modem Approaches to Animal Cell Technology, Ed. Spier et al., Butterworth, U.K., [1987]) as an Eco R1/Bam H1 1.8 Kb fragment) and the Ig heavy chain enhancer (obtained as a PCR amplified fragment using standard procedures described above and human DNA as the template; the oligodeoxynucleotide primer pair is listed in Figure 4) following its digestion with Bgl II and Kpn I. The resultant expression vector was found to lack a small portion of the TK promoter responsible for the transcription of the neomycin gene. This was replaced by insertion into the Eco RI site of a 0.14 kb PCR amplified fragment derived from the CMVIE-AK1-DHFR DNA using the primer pair also listed in Figure 4. The resultant heavy chain expression vector was subsequently modified by removal of the indicated Hind III and Xba I sites. To convert this neomycin selectable vector into one expressing the hygromycin B selectable marker (Figure 11) the neomycin-resistance cassette was removed by digestion first with Eco R1 followed by DNA polymerase-directed fill in of the 5' overhang, then subsequent Sal I digestion. The 1.9 kb hygromycin B expression cassette [TK promoter and TK polyadenylation signal flanking the hygromycin B gene obtained from Gritz and Davies, Gene 25: 179-188 (1983), as the 1.9 kb Bam H1 fragment in plasmid (pLG90)] was removed from the plasmid pAL-2 by Bam H1 digestion and subcloned into the Bam H1 site of the intermediate vector pSP72 (Promega). The hygromycin B cassette was removed from this vector by digestion with Sma I and Sal I and cloned into the expression vector linearized as described above to create a blunt end and Sal I end DNA fragment.

Expression of the 1B4 "veneered" kappa light chain was accomplished by transferring this cistron from its position within the pSP72 intermediate vector to the hygromycin B selectable eukaryotic expression vector (Figure 7). A 1.5 kb DNA fragment resulting from the endonuclease digestion of v1B4 VK/pSP72 intermediate vector with Spe I and ClaI was purified by agarose gel electro-phoresis and ligated into the expression vector which had previously been linearized, by digestion with the same two restriction enzymes and agarose gel purified.

The 1B4 "veneered" heavy chain eukaryotic expression vector was constructed in one step (Figure 9) from an existing vector previously constructed to express a chimaeric form of the 1B4 heavy chain. The "veneered" heavy chain variable region created by PCR amplifcation (Figure 8) was digested with Hind III and Bam H1. The agarose gel purified 0.8 kb fragment was ligated into the Hind III and Bam H1 sites of the pD5/IgH-Enhancer/Neo/1B4 VH-Short Human C-Gamma 4 expression vector following its endonuclease digestion with these two enzymes and subsequent purification by agarose gel electrophoresesis (Figure 9). Transformants containing both variable and constant regions were identified. Plasmid DNAs were grown (Maniatis et al., supra) and purified for transfection into recipient mammalian cells (Maniatis et al., supra; Birbion and Doly, supra.).

Equal amounts (10µg) of the plasmids encoding the "veneered" IgG4 heavy chain and the "veneered" kappa light chain were transfected by standard calcium phosphate precipitation procedures into human 293 cells and african green monkey kidney CV-1P cells. The culture supernatant fluids were assayed by a trapping Elisa (described below) for the secretion of a human kappa light chain containing IgG4 immunoglobulin.

An Elisa was developed for the quantitation of the amounts of a 1B4 recombinant antibody expressed in conditioned mammalian cell growth medium. Immulon-2 (Dynatech Labs.) 96-well plates are coated overnight with a 5µg/ml solution of mouse anti-human k chain constant domain monoclonal antibody (cat. #MC009, The Binding Site, Inc., San Diego, CA) in 0.1M NaHCO₃ buffer (pH 8.2) at 4°C, and blocked with 1% bovine serum (BSA) in 0.1M NaHCO₃ for 1h at 25°C. After this and all subsequent steps, washing was performed with phosphate buffered saline (PBS). The wells are then challenged with conditioned medium containing recombinant anti-CD18 antibody, or with predetermined quantities of human IgG4 purified by protein A Sepharose® (Pharmacia Fine Chemicals) chromatography from human IgG4 myeloma serum (cat. # BP026,The Binding Site, Inc.). All samples are diluted in PBS containing 0.05% Tween®-20. 100µl aliquots are incubated for 1h at 37°C in triplicate, and standard calibration curves are constructed using IgG4 concentrations ranging from 10 ng/ml to 100 ng/ml. Bound and fully assembled human IgG4 (either native or recombinant "veneered"1B4 human IgG4 constructs) are detected with 100µl aliquots of a 1:500 dilution of mouse anti-human IgG4 Fc monoclonal antibody conjugated to alkaline phosphatase (cat #05-3822, Zymed Laboratories, Inc.) in phosphate buffered saline (PBS) containing 1% BSA. After incubation for 1h at 37°C and subsequent washing, the quantities of bound conjugate are detected by incubating all samples with a 1 mg/ml solution of p-nitrophenyl phosphate in 0.1M 2,2'amino-methyl-propanediol buffer, pH 10.3, for 30 min at 25°C. The adsorbance of the wells is determined with a UV Max ELISA plate reader (Molecular Devices) set at 405 nm. All supernatant fluids contain this immunoglobulin, though in various amounts. The antibody secreted by the transfected 293 cells is concentrated by protein A chromatography and the concentrations of the recombinant human "veneered" anti-CD18 antibody determined by the trapping Elisa described above, is used to compete with the binding of radiolabeled murine 1B4 to the CD18 ligand on the surface of activated human PMNs. Affinities of various anti-CD18 antibody constructs are determined using a competitive ¹²⁵I-m1B4 soluble binding assay with stimulated human polymorphonuclear leukocytes (PMNs). Purified murine anti-CD18 monoclonal antibody (50 ug; m1B4) is iodinated using chloramine-T (Hunter, W.M. and Greenwood, F.C., Nature 194: 495-496, 1962), and the radiolabeled antibody purified using a Bio-Sil® TSK250 (Biorad, Richmond, CA) gel filtration HPLC column (which fractionates proteins in the range of 1-300 x 10³ daltons) equilibrated in 0.1M phosphate buffer, pH 7.0. Effluent radioactivity is monitored with an in-line detector (Beckman Model 170; Beckman, Fullerton,CA) and total protein measured at OD₂₈₀ with a Kratos Spectroflow 757 detector (Kratos, Mawah, N.J.). A single ¹²⁵I-m1B4 peak composed of coincident OD₂₈₀ and radioactivity tracings characteristically elutes 6 minutes, 30 seconds following sample injection. Specific activity of the product is generally about 10µCi/µg protein, and 97-99% of the counts are precipitable with 10% trichloroacetic acid. The binding of this radiolabeled antibody is assessed on human PMNs purified on a discontinuous Ficoll/Hypaque gradient (English, D. and Anderson, B.R., J. Immunol. Methods 5: 249-255, 1974) and activated with 100 ng/ml phorbol myristate for 20 minutes at 37°C (Lo et al., J. Exp. Med. 169: 1779-1793, 1989). To determine the avidity of antibodies for CD18 molecules on the PMN surface, about 1 x 105 activated PMNs are incubated in a buffer such as Hanks balanced salt solution containing 20 mM Hepes (pH 7.2), 0.14 units aprotinin (Sigma Chemical Co.) and 2% human serum albumin (binding buffer) containing 1.3 ng ¹²⁵I-m1B4 (2.8 x 10⁻¹¹M) in the presence of increasing concentrations of unlabeled m1B4 antibody (10⁻⁷ to 10⁻¹⁵M) in a 300 ul reaction volume for about 1 h at about 4°C with constant agitation. Cell bound 1B4 is separated from the unbound antibody by centrifugation through a 0.5M sucrose cushion (4,800 x g, 3 minutes); the tubes are frozen on dry ice, and the tips cut off and counted with an LKB gamma counter. The IC₅₀ of the anti-CD18 antibody for the inhibition of ¹²⁵I-m1B4 antibody binding is calculated using a four parameter fitter program (Rodbard, D, Munson, P.J., and DeLean, In, "Radioimmunoassay and Related Procedures in Medicine", International Atomic Energy Agency, Vienna, vol I,469-504, 1978). The affinity of the "veneered" anti-CD18 antibody for the CD18 ligand is determined in a similar manner using murine ¹²⁵I-mlB4 antibody and increasing quantities, as determined by the trapping Elisa, of unlabeled "veneered" anti-CD18 antibody. The results of the binding assays are shown in Figure 13 and indicate that the avidity of the "veneered" heavy chain and light chain recombinant 1B4 antibody is equivalent to that of the murine 1B4 monoclonal antibody.

The "veneered" heavy and light chain expression vectors were co-transfected into CV1P monkey kidney cells using 20 µg of each plasmid to prepare 2 mL of the calcium phosphate precipitated solution. One mL was placed in the medium overlaying each 100 mm dish of CV1P cells. After 4 hr at 37°C the medium was replaced with 1 mL of 15% glycerol in 1 x HBS (Hepes buffered salt). Following the 3 min glycerol shock, 10 mL of PBS as added, the cell monolayers were aspirated, washed once with 10 mL of PBS, and re-fed with fresh medium (DMEM + 10% heat inactivated new born calf serum) containing 200 µg of hygromycin B and 800 µg of G418 per mL. Cloning cylinders (Fishney, In, Culture of Animal Cells, Alan R. Liss, Inc. New York, 1983) were used to isolate individual colonies prior to their expansion and subsequent assay for productivity. Two clones, #11 and #48, were found to express sufficient amounts of v1B4 to warrant their expansion and ultimate accessioning.

## Claims

1. A method of producing an immunoglobulin having the ligand binding properties of an immunoglobulin from a first mammalian species and the immunogenicity of an immunoglobulin from a second mammalian species comprising:
a. comparing a variable domain of an immunoglobulin of the first mammalian species with the variable domains immunoglobulins of the second mammalian species at corresponding framework amino acid sequences;
b. selecting from the variable domains of the second mammalian species the variable domain which is most similar to the first mammalian species variable domain at corresponding framework amino acid sequences;
c. identifying framework amino acid residues of the first mammalian species vaxiable domain which differ from the amino acid residues at the corresponding position of the variable domain selected in section b, said differing amino acid residues being limited to those having a fractional accessibility of at least 0.61 which are not adjacent to a complementarity-determining region;
d. replacing only the amino acid residues identified in section c with the corresponding residues present in the sequence selected in section b;
e. preparing a DNA sequence encoding the immunoglobulin produced by section d;
f. inserting the RNA sequence into a replicable expression vector operably linked to a suitable promoter compatible with a host cell;
g. transforming a host cell with the vector of section f;
h. culturing the host cell; and
i. recovering the immunoglobulin from the host cell culture.

## Patentansprüche

1. Verfahren zur Herstellung eines Immunglobulins mit den Ligandenbindungseigenschaften eines Immunglobulins von einer ersten Säugerspezies und der Immunogenizität eines lmmunglobulins von einer zweiten Säugerspezies, umfassend:
a) Vergleichen einer variablen Domäne eines Immungfobulins der ersten Säugerspezies mit den variablen Domänen von Immunglobulinen der zweiten Säugerspezies bei korrespondierenden Gerüst-Aminosäuresequenzen;
b) Auswählen derjenigen variablen Domäne aus den variablen Domänen der zweiten Säugerspezies, welche der variablen Domäne der ersten Säugerspezies bei korrespondierenden Gerüst-Aminosäuresequenzen am ähnlichsten ist;
c) identifizieren von Gerüst-Aminosäureresten der variablen Domäne der ersten Säugerspezies, weiche sich von den Aminosäureresten an der korrespondierenden Position der in Abschnitt b) ausgewählten variablen Domäne unterscheiden, wobei die unterschiedlichen Aminosäurereste auf diejenigen mit einer relativen Zugänglichkeit (fractional accessibility) von mindestens 0,61, welche nicht neben einer komplementaritäts-bestimmenden Region liegen, beschränkt sind;
d) Ersetzen nur derjenigen Aminosäurereste, welche in Abschnitt c) identifiziert wurden, durch die korrespondierenden Reste, welche in der in Abschnitt b) ausgewählten Sequenz vorliegen;
e) Herstellen einer DNA-Sequenz, welche für das gemäß Abschnitt d) hergestellte Immunglobulin kodiert;
f) Einfügen der DNA-Sequenz in einen replizierbaren Expressionsvektor, welcher funktionsfähig mit einem geeigneten Promoter, der mit einer Wirtszelle kompatibel ist, verknüpft ist;
g) Transformieren einer Wirtszelle mit dem Vektor von Abschnitt f);
h) Kultivieren der Wirtszelle, und
i) Gewinnen des Immunglobulins aus der Wirtszellkultur.

## Revendications

1. Procédé de production d'une immunoglobuline ayant les propriétés de liaison à un ligand d'une immunoglobuline d'une première espèce de mammifère et le caractère immunogène d'une immunoglobuline d'une deuxième espèce de mammifère, comprenant :
a. la comparaison d'un domaine variable d'une immunoglobuline de la première espèce de mammifère avec les domaines variables des immunoglobulines de la deuxième espèce de mammifère au niveau séquences des acides aminés dans les régions de base correspondante ;
b. la sélection parmi les domaines variables de la deuxième espèce de mammifère, du domaine variable qui est le plus similaire au domaine variable de la première espèce de mammifère au niveau séquences des acides aminés dans les régions de base correspondante ;
c. l'identification des résidus acide aminé en cadre du domaine variable de la première espèce de mammifère, qui diffèrent des résidus acide aminé en la position correspondante du domaine variable choisi à la partie b., lesdits résidus acide aminé différents étant limités à ceux ayant une accessibilité fractionnelle d'au moins 0,61, qui ne sont pas adjacents à une région déterminant la complémentarité ;
d. le remplacement de seuls les résidus acide aminé identifiés à la section c- par les résidus correspondants présents dans la séquence sélectionnée à la partie b. ;
e. la préparation d'une séquence d'ADN codant l'immunoglobuline produite à la partie d. ;
f. l'insertion de la séquence d'ADN dans un vecteur d'expression réplicable, lié de manière fonctionnelle à un promoteur approprié compatible à une cellule hôte ;
g. la transformation d'une cellule hôte avec le vecteur de la partie f. ;
h. la mise en culture de la cellule hôte, et
i. la récupération de l'immunoglobuline depuis la culture de la cellule hôte.
